(19) Europäisches Patentamt
European Patent Office
Office européen des brevets

(11) **EP 0 790 499 B1**

(12) **EUROPÄISCHE PATENTSCHRIFT**

(45) Veröffentlichungstag und Bekanntmachung des Hinweises auf die Patenterteilung:
**14.11.2007** **Patentblatt 2007/46**

(51) Int Cl.:
*G01N 33/497* *(2006.01)* *G01N 33/00* *(2006.01)*
*A61B 5/00* *(2006.01)*

(21) Anmeldenummer: **97101979.9**

(22) Anmeldetag: **07.02.1997**

(54) **Verfahren zur Eichung von Gasmesssensoren für gelöste Gase und Verfahren zur Konzentrationsmessung von CO2 in Blut mit Hilfe eines solchen Eichverfahrens**

Method for calibrating dissolved gas sensors and method for measuring the CO2 concentration in blood with such calibrating method

Méthode de calibration de détecteurs de gaz dissolus et méthode de mesure de la concentration du CO2 dans le sang à l'aide d'une telle méthode de calibration

(84) Benannte Vertragsstaaten:
**CH DE ES FR GB IT LI NL SE**

(30) Priorität: **13.02.1996 DE 19605246**

(43) Veröffentlichungstag der Anmeldung:
**20.08.1997 Patentblatt 1997/34**

(73) Patentinhaber: **SPHERE Medical Limited Cambridge Cambridgeshire CB2 5GG (GB)**

(72) Erfinder:
• **Gumbrecht, Walter, Dr.**
  **91074 Herzogenaurach (DE)**
• **Stanzel, Manfred, Dr.**
  **91088 Bubenreuth (DE)**

(74) Vertreter: **Gillard, Richard Edward et al Elkington and Fife LLP Prospect House 8 Pembroke Road Sevenoaks Kent TN13 1XR (GB)**

(56) Entgegenhaltungen:
EP-A- 0 454 033    EP-A- 0 560 353
WO-A-94/14323    US-A- 3 681 255
US-A- 4 221 567    US-A- 4 700 560
US-A- 5 061 631    US-A- 5 330 634

• GUMBRECHT W ET AL: "SENSORSYSTEM FOR ONLINE BLOODMONITORING" PROCEEDINGS OF THE ANNUAL INTERNATIONAL CONFERENCE OF THE ENGINEERING IN MEDICINE AND BIOLOGY SOCIETY. PARIS, OCT. 29 - NOV. 1, 1992, NEW YORK, IEEE, US, Bd. 6 CONF. 14, 29. Oktober 1992 (1992-10-29), Seite 2713 XP000347086

**Beschreibung**

Die Erfindung betrifft ein Verfahren zur Eichung eines Sensors zur Messung der Konzentration eines in einer Flüssigkeit gelösten Gases nach dem Oberbegriff des Patentanspruchs 1 und ein Verfahren zur Messung der Konzentration von in Blut gelöstem $CO_2$ mit Hilfe eines derartigen Eichverfahrens.

Beispiele für Sensoren zur Messung der Konzentration eines in einer Flüssigkeit gelösten Gases sind chemische Sensoren zur Messung der Konzentration von Blutgasen, insbesondere von in Blut gelöstem $CO_2$, $O_2$ und/oder $N_2$.

Bei Sensoren zur Messung der Konzentration eines in einer Flüssigkeit gelösten Gases können unter anderem beispielsweise

a) Sensoren, die einen zu einem Logarithmus der Konzentration des in der Flüssigkeit gelösten Gases proportionalen Meßwert und

b) Sensoren, die einen zur Konzentration des in der Flüssigkeit gelösten Gases direkt proportionalen Meßwert erzeugen, unterschieden werden.

Ein Beispiel für einen Sensor nach a) ist ein potentiometrischer chemischer Sensor zur Messung der Konzentration von in Blut gelöstem $CO_2$, der einen der $CO_2$-Konzentration entsprechenden Meßwert in Form einer elektrischen Spannung erzeugt, die zum Logarithmus der gemessenen $CO_2$-Konzentration proportional ist.

Ein Beispiel für einen Sensor nach b) ist ein amperometrischer chemischer Sensor zur Messung der Konzentration von $O_2$ in Blut, der einen der gemessenen $O_2$-Konzentration entsprechenden Meßwert in Form eines elektrischen Stroms erzeugt, dessen Stärke zur gemessenen $O_2$-Konzentration direkt proportional ist.

Ein Maß für die Konzentration eines in einer Flüssigkeit gelösten Gases ist bekanntermaßen der Partialdruck des in dieser Flüssigkeit gelösten Gases und es ist zumindest in der Medizin üblich, die Konzentration eines gelösten Gases durch diesen Druck anzugeben. In bezug auf in Flüssigkeiten gelöste oder in Gasphasen enthaltene Gase werden im folgenden die Begriffe "Konzentration" und "Partialdruck" dort, wo es auf eine Unterscheidung nicht wesentlich ankommt, synonym verwendet. Wo es auf eine Unterscheidung ankommt, wird darauf hingewiesen.

Um den von den Sensoren gelieferten Meßwerten die richtigen Konzentrationen bzw. Partialdrucke zuordnen zu können, müssen sie geeicht und ggf. von Zeit zu Zeit nachgeeicht werden. Zur Eichung werden dem betreffenden Sensor zumindest zwei jeweils das Gas in genau definierter aber voneinander verschiedener Konzentration gelöst enthaltende Eichflüssigkeiten zugeführt und die diesen verschiedenen Konzentrationen bzw. Partialdrucken entsprechenden Meßwerte gewonnen.

Die Herstellung der Eichflüssigkeiten mit jeweils genau definierter Konzentration des gelösten Gases erfolgt üblicherweise so, daß die verschiedenen Eichflüssigkeiten getrennt voneinander hergestellt und getrennt dem Sensor zur Konzentrationsmessung zugeführt werden.

Zur Herstellung der verschiedenen Eichflüssigkeiten bedienen sich verschiedene Hersteller klinischer Analysegeräte (beispielsweise die Firmen Ciba-Corning, Instrumentation Laboratory, NOVA, Radiometer oder AVL) bei Geräten zur Messung der Konzentration von Blutgasen im Blut verschiedener Verfahren:

- Aus zwei Druckgaszylindern, die voneinander verschiedene, definierte und beispielsweise aus $CO_2$, $O_2$ und/oder $N_2$ bestehende Gasgemische enthalten, werden die Gemische entnommen und im befeuchteten Zustand über den betreffenden Sensor geleitet, wobei die Eichflüssigkeiten durch die befeuchteten Gasgemische gegeben sind.

- Aus zwei Druckgaszylindern, die beispielsweise synthetische Luft und $CO_2$ enthalten, werden mittels eingebauter Gasmischapparatur verschiedene definierte Gasgemische hergestellt und im befeuchteten Zustand über den betreffenden Sensor geleitet, wobei die Eichflüssigkeiten durch die befeuchteten Gasgemische gegeben sind.

- Aus einer Druckgasflasche wird $CO_2$ entnommen und in einer Gasmischapperatur mit Raumluft vermischt, so daß verschiedene Gasgemische mit voneinander verschiedenen $CO_2$-Konzentrationen zur Verfügung stehen, aus denen durch Einleiten je eines Gasstroms in je eine pH-Pufferlösung und Tonometrieren verschiedene Eichflüssigkeiten hergestellt werden können.

Zwei Hersteller von intra-arteriellen Blutgasüberwachungssystemen (Puritan Bennet, Paratrend 7) verwenden ein Verfahren, bei dem zur einmaligen Eichung dieser Systeme vor deren Inbetriebnahme verschiedene Gasmischungen aus zwei bis drei Druckgaszylindern entnommen und durch Eichküvetten geleitet werden.

Einen anderen Weg beschreitet die Firma Mallinkrot, die auf Druckgasflaschen ganz verzichtet und zwei tonometrierte Eichflüssigkeiten in metallbeschichteten, gasundurchlässigen Kunststoffbeuteln für bestimmte Analysegeräte dieser Firma bereitstellt.

US-A-4,221,567 offenbart ein Verfahren zur Eichung von Gasmesssonsoren für gelöste Gase und Verfahren zur Konzentrationsmessung von CO2 in Blut mit Hilfe eines solchen Eichverfahrens, wobei der Sensor durch eine Zweipunktmessung mit $O_2$ und $CO_2$ geeicht wird.

Der Erfindung liegt die Aufgabe zugrunde, ein Eichverfahren der eingangs genannten Art bereitzustellen, welches gegenüber den bekannten Eichverfahren dieser Art einfacher durchführbar ist.

Diese Aufgabe wird durch die im kennzeichnenden Teil des Patentanspruchs 1 angegebenen Merkmale gelöst.

Ein Vorteil des erfindungsgemäßen Verfahrens ist darin zu sehen, daß nur eine Eichflüssigkeit, die vorbestimmte, auf herkömmliche Weise hergestellt werden muß. Dies führt vorteilhafterweise dazu, daß die ein solches Eichverfahren verwendenden Analysegeräte vereinfacht, verkleinert und verbilligt werden können.

Die Änderung der Konzentration des in der vorbestimmten Eichflüssigkeit gelösten Gases auf die vorbestimmte andere Konzentration durch einen Stofftransport zwischen der vorbestimmten Eichflüssigkeit und einer vorbestimmten Gasphase erzeugt.

Die zu verwendende Gasphase kann in weitesten Grenzen frei gewählt werden und unterliegt nur der Beschränkung, daß der Stofftransport, beispielsweise Gasaustausch, zwischen der vorbestimmten Eichflüssigkeit und dieser Gasphase eine Änderung der Konzentration des in dieser Eichflüssigkeit gelösten Gases herbeiführen muß.

Bevorzugterweise wird dazu das im Anspruch 2 angegebene Verfahren verwendet, bei welchem in dem Fall, daß der Partialdruck des Gases in der Gasphase größer als in der vorbestimmten Eichflüssigkeit ist, der Partialdruck des Gases in dieser Eichflüssigkeit durch Stofftransport zwischen der Gasphase und der Eichflüssigkeit erhöht, und in dem Fall, daß der Partialdruck des Gases in der Gasphase kleiner als in der vorbestimmten Eichflüssigkeit ist, der Partialdruck des Gases in dieser Eichflüssigkeit durch Stofftransport zwischen der Gasphase und der Eichflüssigkeit erniedrigt wird.

Dies ist ein Fall, bei welchem zwischen Partialdruck und Konzentration zu unterscheiden ist, da die Konzentration und der Partialdruck bei einem in einer Flüssigkeit gelösten Gas mit einem anderen Proportionalitätsfaktor zusammenhängen als bei einem in einer Gasphase enthaltenen Gas. Der Stofftransport zwischen Flüssigkeit und Gasphase aufgrund eines Partialdrukkunterschiedes kommt zum Erliegen, wenn sich die Partialdrükke des Gases in der Gasphase und in der Flüssigkeit einander angeglichen haben. In diesem Zustand ist aber wegen der voneinander verschiedenen Proportionalitätsfaktoren von Gasphase und Flüssigkeit die Konzentration des Gases in der Gasphase eine andere als in der Flüssigkeit. Jedoch überall dort, wo die Proportionalitätsfaktoren gleich oder miteinander vergleichbar sind, so daß sie nicht unterschieden werden brauchen, beispielsweise bei einem in zwei ähnlichen Flüssigkeiten gelösten oder in zwei ähnlichen Gasphasen enthaltenen Gas, muß auch zwischen Konzentration und Partialdruck nicht unterschieden werden. In diesem letztgenannten Fall steht im folgenden p sowohl für Partialdruck als auch für Konzentration. Die Konzentration von in Flüssigkeit gelösten nichtflüchtigen Stoffen ist mit c bezeichnet.

Die Gasphase kann ein Gasgemisch sein, in welchem das in der vorbestimmten Eichflüssigkeit gelöste Gas nicht oder anteilmäßig mit höherem oder niedrigerem Partialdruck als in der der vorbestimmten Eichflüssigkeit enthalten ist.

Bevorzugterweise wird eine aus Luft bestehende Gasphase verwendet (Anspruch 3), die vorteilhafterweise die Raumluft ist, in der das betreffende Analysegerät steht, weil dann keine Maßnahmen oder Behälter zur Bereitstellung der Gasphase erforderlich sind, die eine Komplizierung und Verteuerung des betreffenden Geräts mit sich bringen.

Es kann auch eine Gasphase verwendet werden, die nur aus dem in der vorbestimmten Eichflüssigkeit gelösten Gas besteht, wobei auch hier je nach dem Druckunterschied zwischen dem Druck des die Gasphase bildenden Gases und dem Partialdruck dieses in der vorbestimmten Eichflüssigkeit gelösten Gases durch Stofftransport der Partialdruck bzw. die Konzentration des Gases in dieser Eichflüssigkeit erhöht oder erniedrigt wird.

Es können auch Gasphasen verwendet werden, bei denen durch den Stofftransport die Konzentration bzw. der Partialdruck des in der Eichflüssigkeit gelösten Gases aufgrund eines anderen Wirkungsmechanismus als nur durch Partialdruckausgleich allein geändert wird. Beispielsweise kann aufgrund des Stofftransports bzw. Gasaustauschs in der vorbestimmten Eichflüssigkeit eine chemische Reaktion auslöst werden, die das in dieser Flüssigkeit gelöste Gas teilweise zum Verschwinden bringt und dadurch die Konzentration bzw. den Partialdruck dieses Gases in dieser Eichflüssigkeit verringert, oder dieses Gas zusätzlich entstehen läßt und dadurch die Konzentration bzw. den Partialdruck dieses Gases in dieser Eichflüssigkeit vergrößert.

Ein besonderer Vorteil des erfindungsgemäßen Verfahrens wird aus der im Anspruch 4 angegebenen Verfahrensdurchführung ersichtlich. Diese Verfahrensdurchführung zeigt einerseits, daß bei dem erfindungsgemäßen Verfahren die Eichflüssigkeiten durch herkömmliche und billige gasdurchlässige Schläuche den Sensoren zugeführt werden können, andererseits, daß zur Herstellung der Eichflüssigkeit mit der vorbestimmten anderen, von der Konzentration des Gases der vorbestimmten Eichflüssigkeit verschiedenen Konzentration dieses Gases keinerlei besonderen, insbesondere baulichen Maßnahmen erforderlich sind, sondern daß die Unzulänglichkeit der Gasdurchlässigkeit der billigen Schläuche vorteilhafterweise geradezu Vorraussetzung für die Verfahrensdurchführung ist. Darüber hinaus besteht der Vorteil, daß sowohl die vorbestimmte Eichflüssigkeit als auch die von dieser Eichflüssigkeit erzeugte Eichflüssigkeit der vorbestimmten anderen Konzentration dem betreffenden Sensor in ein und demselben Schlauch zugeführt wird, so daß zum Zuführen der beiden Eichflüssigkeiten zum Sensor vorteilhafterweise ein einziger Schlauch genügt.

Bevorzugterweise wird die vorbestimmte Eichflüssigkeit einem gasundurchlässigem Gefäß entnommen (Anspruch 5). An dieses Gefäß kann der Schlauch zum Zuführen der Eichflüssigkeiten zum Sensor angeschlossen werden. Das Gefäß kann ein gasundurchlässiger Beutel sein, der vorzugsweise ein metallisierter Kunststoffbeutel ist. Die vorbestimmte Eichflüssigkeit kann dem Gefäß mittels einer Pumpe, beispielsweise einer Dosierpumpe entnommen werden.

Das erfindungsgemäße Verfahren ist, obgleich nicht darauf beschränkt, sowohl bei Sensoren, die einen zu einem Logarithmus der Konzentration bzw. des Partialdrucks des in der Flüssigkeit gelösten Gases proportionalen Meßwert erzeugen (Anspruch 6), als auch bei Sensoren, die einen zur Konzentration bzw. zum Partialdruck des in der Flüssigkeit gelösten Gases direkt proportionalen Meßwert erzeugen (Anspruch 7), anwendbar.

Vielfach ist es so, daß die Konzentration bzw. der Partialdruck des in einer Eichflüssigkeit gelösten Gases eine definierte, nichtkonstante Funktion des pH-Wertes dieser Eichflüssggikeit ist. Eine derartige vorbestimmte Eichflüssigkeit spielt beispielsweise bei einem später beschriebenen Verfahren zur Bestimmung der $CO_2$-Konzentration von Blut eine Rolle.

In einem derartigen Fall ist es generell zweckmäßig, wenn gemäß Anspruch 8 die vorbestimmte andere Konzentration der von der vorbestimmten Eichflüssigkeit beispielsweise durch Stofftransport bzw. Gasaustausch zwischen der Gasphase und der vorbestimmten Eichflüssigkeit erzeugten Eichflüssigkeit durch Ermitteln des pH-Wertes der so erzeugten Eichflüssigkeit bestimmt wird.

In diesem Zusammenhang ist es in besonderen Fällen möglich, gemäß Anspruch 9 eine vorbestimmte Eichflüssigkeit zu verwenden, bei welcher ein Logarithmus der Konzentration des in dieser Eichflüssigkeit gelösten Gases eine nichtkonstante lineare Funktion f des variablen pH-Wertes dieser Eichflüssigkeit ist. Ein Beispiel für eine derartige Eichflüssigkeit ist eine später beschriebene wäßrige Lösung, in der $CO_2$ und Bikarbonat, d.h. $HCO_3^-$, gelöst sind, und die neben diesem Puffersystem keine weiteren pH-puffernden Substanzen mehr enthält.

Im Fall des Anspruchs 9, bei welchem der Sensor einen zu einem Logarithmus der Konzentration des in der Flüssigkeit gelösten Gases proportionalen Meßwert erzeugt, wird ein Verfahren nach Anspruch 7 oder 8 zweckmäßigerweise so ausgeführt, wie es im Anspruch 10 angegeben ist.

Das erfindungsgemäße Verfahren ist prinzipiell für alle gelösten Gase anwendbar, für die es Sensoren gibt, mit denen die Konzentration bzw. der Partialdruck dieser gelösten Gase gemessen werden kann. Beispiele für Sensoren, die einen zum Logarithmus der Gaskonzentration proportionalen Meßwert erzeugen, sind neben dem Sensor zur Messung der Konzentration von in Flüssigkeit gelöstem $CO_2$ Sensoren zur Messung der Konzentration von in Flüssigkeit gelöstem $NH_3$, $SO_2$, $NO_2$ oder $H_2S$.

Von besonderer Bedeutung sind Gase, die in einer physiologischen Körperflüssigkeit oder in einem physiologischen Gewebe gelöst sind, darunter in Blut gelöstes $CO_2$. Auf diese besonderen Fälle sind die erfindungsgemäßen Verfahren nach den Ansprüchen 11 bis 14 gerichtet.

Das erfindungsgemäße Eichverfahren nach Anspruch 11 und 13 oder 14 ist besonders vorteilhaft bei einem Verfahren zur Messung der Konzentration von in Blut gelöstem $CO_2$ mit einem $CO_2$-Sensor zum Messen der $CO_2$-Konzentration und Erzeugen eines zum Logarithmus der $CO_2$-Konzentration proportionalen Meßwertes anwendbar. Der Anspruch 15 ist auf ein derartiges erfindungsgemäßes Verfahren zur Konzentrationsmessung von $CO_2$ in Blut gerichtet, das auf der folgenden Problematik basiert:

Im Blut gelöste Stoffe, darunter die Blutgase $N_2$, $O_2$ und $CO_2$ und nichtflüchtige andere Stoffe, darunter $K^+$, $Ca^{2+}$ und Biomoleküle wie Glukose, Lactat usw., und erforderlichenfalls der pH-Wert des Blutes, werden durch diesen Stoffen zugeordnete Sensoren gemessen, wobei eine auch Baseline-Flüssigkeit genannte und in einem vorzugsweise gasdichten Gefäß enthaltene, vorzugsweise pH-gepufferte Grundlinienflüssigkeit und eine in einem anderen gasdichten Gefäß enthaltene Eichflüssigkeit verwendet werden, welche die zu messenden Blutstoffe in jeweils möglichst genau definierter physiologischer Konzentration gelöst enthalten und erforderlichenfalls einen jeweils möglichst genau definierten physiologischen pH-Wert aufweisen.

Die physiologische Konzentration eines Stoffes bedeutet jede Konzentration, in der dieser Stoff in einem lebenden Organismus vorkommt, sei es in einer Körperflüssigkeit oder im Gewebe des Organismus und/oder sei es im gesunden oder kranken Organismus. Entsprechend bedeutet der physiologische pH-Wert jeden pH-Wert, der im lebenden Organismus vorkommt, sei es in einer Körperflüssigkeit oder im Gewebe des Organismus und/oder sei es im gesunden oder kranken Organismus.

Die physiologische Konzentration von $CO_2$ im Blut eines Menschen liegt beispielsweise zwischen einem Partialdruck von 10 Torr und 150 Torr, wobei 1 Torr gleich 133,3224 Pa und annähernd gleich 1 mmHg ist. Der physiologische pH-Wert dieses Blutes liegt zwischen 6,8 und 7,8.

Die normalphysiologische Konzentration eines Stoffes bedeutet jede im gesunden Organismus normalerweise vorkommende physiologische Konzentration dieses Stoffes. Entsprechend bedeutet der normalphysiologische pH-Wert jeden im gesunden Organismus normalerweise vorkommenden physiologischen pH-Wert.

Die normalphysiologische Konzentration von $CO_2$ im Blut eines Menschen liegt beispielsweise zwischen einem Partialdruck von 35 Torr und 45 Torr. Der normalphysiologische pH-Wert dieses Blutes liegt zwischen 7,35 und 7,45.

Zur Messung werden jedem Sensor beispielsweise abwechselnd die genannte Grundlinienflüssigkeit aus ihrem Gefäß und das beispielsweise arterielle Blut zugeführt, wobei dieser Sensor die Konzentration bzw. Partialdruck des ihn betreffenden Stoffes in der Grundlinienflüssigkeit und im Blut mißt.

Jeder Sensor wird zusätzlich mit Hilfe der Eichflüssigkeit geeicht, in der die in der Grundlinienflüssigkeit enthaltenen Stoffe in einer von der Grundlinienflüssigkeit verschiedenen Konzentration gelöst sein müssen und die erforderlichenfalls einen vom pH-Wert der Grundlinienflüssigkeit verschiedenen pH-Wert aufweisen muß. Nach erstmaliger Eichung kann von Zeit zu Zeit eine Nacheichung mit dieser Eichflüssigkeit vorgenommen werden.

Ein Problem tritt auf, wenn die Konzentration bzw. der Partialdruck von in Blut gelöstem $CO_2$ gemessen werden soll und als Grundlinienflüssigkeit eine unter Normalbedingung, d.h. bei Raumtemperatur und normalem Luftdruck luftgesättigte wässrige Lösung verwendet wird. $CO_2$ ist in der Luft unter Normalbedingung mit einem Partialdruck von weniger als 1 Torr enthalten. Die mit dieser Luft gesättigte Grundlinienflüssigkeit enthält das $CO_2$ in einer Konzentration gelöst, die gleich dem weniger als 1 Torr betragenden Partialdruck des $CO_2$ in der Luft ist. Diese $CO_2$-Konzentration der Grundlinienflüssigkeit liegt damit weit unterhalb der bei einem Partialdruck von etwa 10 Torr liegenden unteren Grenze der physiologischen Konzentration von $CO_2$ in Blut. Darüberhinaus ist die $CO_2$-Konzentration der luftgesättigten Grundlinienflüssigkeit in der Regel nicht ausreichend genau bekannt.

Aus diesen Gründen kann eine solche luftgesättigte Grundlinienflüssigkeit nicht zur Eichung des $CO_2$-Sensors herangezogen werden.

Um den $CO_2$-Sensor in diesem Fall dennoch eichen zu können, müßte neben der Eichflüssigkeit eine zweite Eichflüssigkeit verwendet werden, welche das $CO_2$ in einer genau definierten, aber von der einen Eichflüssigkeit deutlich verschiedenen physiologischen Konzentration gelöst enthält und die erforderlichenfalls einen von der einen Eichflüssigkeit deutlich verschiedenen aber genau definierten physiologischen pH-Wert aufweist. Diese zweite Eichflüssigkeit müßte in einem zusätzlichen gasdichten Gefäß enthalten sein, wodurch die Komplexität und damit die Kosten für das $CO_2$-Meßgerät in nicht akzeptabler Weise erhöht würde.

Das im Anspruch 15 angegebene erfindungsgemäße Verfahren zur Messung der $CO_2$-Konzentration in Blut benötigt, da die zweite benötigte Eichflüssigkeit auf einfache Weise von selbst aus der einen, d.h. der vorbestimmten Eichflüssigkeit gewonnen wird, neben dem gasdichten Gefäß für die Grundlinienflüssigkeit vorteilhafterweise nur noch das gasdichte Gefäß für die vorbestimmte Eichflüssigkeit, so daß vorteilhafterweise eine bereits vorgeschlagene Vorrichtung zur Durchführung eines Verfahrens zur Messung der Konzentration von in Blut gelösten Stoffen mit Hilfe einer Grundlinienflüssigkeit und einer Eichflüssigkeit ohne Modifikationen verwendet werden kann.

Bevorzugte und vorteilhafte Ausgestaltungen des Verfahrens nach Anspruch 15 gehen aus den Ansprüche 16 bis 23 hervor.

Die Erfindung ist insbesondere gut für Online-Blutgas-Über wachungssysteme geeignet.

Die Erfindung wird in der nachfolgenden Beschreibung anhand der Figuren beispielhaft näher erläutert. Es zeigen

Figur 1     in schematischer Darstellung eine bereits vorgeschlagene Vorrichtung zur Durchführung eines Verfahrens zur Messung der Konzentration von in Blut gelösten Stoffen mit Hilfe einer Grundlinienflüssigkeit und einer Eichflüssigkeit,

Figur 2     die Differenz $\Delta U_{pCO2}$ zwischen einem an einer zu analysierenden Flüssigkeit gemessenen Meßwert $U_{pCO2}$ des $CO_2$-Sensors und dem an der Grundlinienflüssigkeit gemessenen Meßwert $U_{pCO2}(BF)$ des $CO_2$-Sensors in Abhängigkeit vom Logarithmus $log(pCO_2)$ des Partialdrucks $pCO_2$ des in der zu analysierenden Flüssigkeit gelösten $CO_2$,

Figur 3     die Differenz $\Delta U_{pH}$ zwischen einem an einer zu analysierenden Flüssigkeit gemessenen Meßwert $U_{pH}$ des pH-Sensors und dem an der Grundlinienflüssigkeit gemessenen Meßwert $U_{pH}(BF)$ des pH-Sensors in Abhängigkeit vom pH-Wert der zu analysierenden Flüssigkeit,

Figur 4     eine bei Durchführung des erfindungsgemäßen Verfahrens zur Messung der $CO_2$-Konzentration in Blut mit der Vorrichtung nach Figur 1 vom $CO_2$-Sensor aufgenommene Meßkurve, die den zeitlichen Verlauf des Meßwerts $U_{pCO2}$ dieses Sensors zeigt, und

Figur 5     eine bei Durchführung des erfindungsgemäßen Verfahrens zur Messung der $CO_2$-Konzentration in Blut mit der Vorrichtung nach Figur 1 vom pH-Sensor aufgenommene Meßkurve, die den zeitlichen Verlauf des Meßwerts $U_{ph}$ dieses Sensors zeigt.

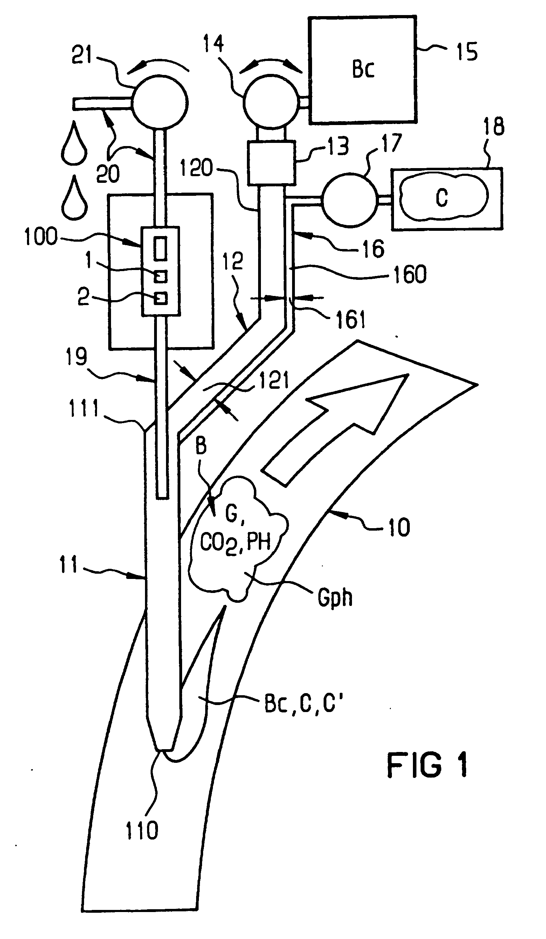

Die in Figur 1 dargestellte bereits vorgeschlagene beispielhafte Vorrichtung weist eine in ein Blutgefäß 10, beispielsweise eine Arterie, eingeführte Kanüle 11 mit einer innerhalb des Blutgefäßes 10 im darin strömenden Blut B befindlichen offenen Spitze 110 und einem außerhalb des Blutgefäßes 10 befindlichen offenen Ende 111 auf.

**EP 0 790 499 B1**

Das Ende 111 ist durch einen eine gasdurchlässige Schlauchwand 120 aufweisenden Schlauch 12 und eine Pumpe 14 mit einem Grundlinienflüssigkeit BF enthaltenden gasdichten Gefäß 15 und durch einen eine gasdurchlässige Schlauchwand 160 aufweisenden Schlauch 16 über eine Pumpe 17 mit einem die vorbestimmte Eichflüssigkeit C enthaltenden gasdichten Gefäß 18 verbunden.

Vom Inneren der Kanüle 11 führt ein Flüssigkeitskanal 19, beispielsweise ebenfalls ein gasdurchlässiger Schlauch, zu einer Sensoreinrichtung 100, die zumindest den $CO_2$-Sensor 1 aufweist. Die Sensoreinrichtung 100 kann aber neben dem $CO_2$-Sensor 1 einen oder mehrere weitere Sensoren zur Messung des Partialdrucks bzw. der Konzentration bestimmter weiterer im Blut B enthaltener flüchtiger und/oder nichtflüchtiger Stoffe, beispielsweise für die weiter unten aufgeführten Stoffe $O_2$, $K^+$, $Ca^{2+}$, Lactat usw., und/oder einen pH-Sensor 2 aufweisen.

Von der Sensoreinrichtung 100 führt ein Flüssigkeitskanal 20 über eine Pumpe 21 zu einem nicht dargestellten Aufnahmegefäß für von den Sensoren gemessener Flüssigkeit, die entsorgt werden kann.

Zur Messung der Konzentration eines von $CO_2$ verschiedenen Gases G, nichtflüchtigen Stoffes oder des pH-Wertes pH(B) des Blutes B wird dem diesem Gas G, nichtflüchtigen Stoff oder pH-Wert zugeordneten Sensor in der Sensoreinrichtung 100 abwechselnd die Grundlinienflüssigkeit BF aus dem Gefäß 15 und das Blut B aus dem Blutgefäß 10 zugeführt.

Die Zufuhr der Grundlinienflüssigkeit BF zum betreffenden Sensor erfolgt so, daß diese Flüssigkeit BF von der Pumpe 14 aus dem Gefäß 15 gesaugt und durch und den gasdurchlässigen Schlauch 12 in die Kanüle 11 gepumpt wird, so daß sich die Kanüle 11 vollständig mit der Grundlinienflüssigkeit BF füllt, wobei in der Kanüle 11 enthaltenes Blut B aus der Kanüle 11 durch deren Spitze 110 in das Blutgefäß 10 verdrängt wird.

Aus der nur noch mit der Grundlinienflüssigkeit BF gefüllten Kanüle 11 wird diese Flüssigkeit BF von der Pumpe 21 durch den Flüssigkeitskanal 19 gesaugt und zur Sensoreinrichtung 100 transportiert, in welcher die genau definierte Konzentration des in der Grundlinienflüssigkeit BF enthaltenen Gases G oder nichtflüchtigen Stoffes oder der genau definierte pH-Wert dieser Flüssigkeit BF vom zugeordneten Sensor gemessen wird.

Die in der Sensoreinrichtung 100 gemessene Grundlinienflüssigkeit BF wird von der Pumpe 21 durch den Flüssigkeitskanal 20 zum Aufnahmegefäß transportiert und dort beispielsweise zur Entsorgung aufgefangen.

Das Blut B wird der Sensoreinrichtung 100 bei abgesperrter Zufuhr von Grundlinienflüssigkeit BF aus dem Gefäß 15 in den Schlauch 12 dadurch zugeführt, daß von der Pumpe 14 Blut B aus dem Blutgefäß 10 in die Kanüle 11 durch deren offene Spitze 110 gesaugt wird, bis die Kanüle 11 vollständig mit Blut B gefüllt ist, und daß von der Pumpe 21 das Blut B aus der blutgefüllten Kanüle 11 durch den Flüssigkeitskanal 19 zur Sensoreinrichtung 100 gefördert wird, in der die Konzentration des Gases G oder nichtflüchtigen Stoffes und/oder der pH-Wert des Blutes B durch den jeweils zugeordneten Sensor gemessen wird.

Das in der Sensoreinrichtung 100 gemessene Blut B wird von der Pumpe 21 durch den Flüssigkeitskanal 20 zum Aufnahmegefäß transportiert und dort beispielsweise zur Entsorgung aufgefangen.

Zu einer Eichung des betreffenden Sensors wird durch die Pumpe 17 eine vorbestimmte Eichflüssigkeit C aus dem Gefäß 18 gesaugt und durch den gasdurchlässigen Schlauch 16 in die Kanüle 11 transportiert, bis die Kanüle 11 vollständig mit der vorbestimmten Eichflüssigkeit C gefüllt und in der Kanüle 11 enthaltenes Blut B oder enthaltene Grundlinienflüssigkeit BF durch deren Spitze 110 in das Blutgefäß 10 verdrängt ist.

Aus der nur noch mit der vorbestimmten Eichflüssigkeit C gefüllten Kanüle 11 wird diese Flüssigkeit C von der Pumpe 21 durch den Flüssigkeitskanal 19 gesaugt und zur Sensoreinrichtung 100 transportiert, in welcher die genau definierte Konzentration des in der Eichflüssigkeit C enthaltenen Gases G oder nichtflüchtigen Stoffes oder der genau definierte pH-Wert dieser Flüssigkeit C vom jeweils zugeordneten Sensor gemessen wird.

Die in der Sensoreinrichtung 100 gemessene Eichflüssigkeit C wird von der Pumpe 21 durch die Rohrleitung 20 zum Aufnahmegefäß transportiert und dort beispielsweise zur Entsorgung aufgefangen.

Ist die Konzentration von $CO_2$ im Blut B zu messen, wird erfindungsgemäß die Gasdurchlässigkeit der Schlauchwände 120, 160 der beiden Schläuche 12 und 16, insbesondere die Gasdurchlässigkeit der Schlauchwand 160 ausgenutzt. Diese gasdurchlässigen Schlauchwände 12 und 16 ermöglichen einen Gasaustausch oder Stofftransport zwischen der im Schlauch 12 bzw. 16 enthaltenen Flüssigkeit und einem die Schlauchwand 120 bzw. 160 außen umgebenden Medium, beispielsweise die Gasphase Gph, der zu einer gewünschten Änderung der Konzentration bzw. des Partialdrucks des $CO_2$ oder eines davon verschiedenen Gases G führt, das in einer im Schlauch 12 bzw. 16 enthaltenen Flüssigkeit gelöst ist.

Als umgebende Gasphase Gph wird vorteilhafterweise Raumluft verwendet, d.h. die Luft des Raumes, in der das Meßgerät steht. Bei Raumtemperatur, d.h. bei 20 bis 23°C, und Normalluftdruck auf normaler Höhe, d.h. bei 720 bis 760 Torr, enthält die Raumluft das $CO_2$ mit einem Partialdruck von weniger als 1 Torr.

Als Grundlinienflüssigkeit BF wird eine luftgesättigte Lösung verwendet, in welcher das $CO_2$ ähnlich wie in der Raumluft Gph mit einem nicht notwendig genau bekannten Partialdruck $pCO_2(BF)$ von weniger als 10 Torr gelöst ist.

Dagegen wird eine Eichflüssigkeit C verwendet, in der $CO_2$ mit einem genau definierten physiologischen Partialdruck $pCO_2(C)$ gelöst ist.

Die Grundlinienflüssigkeit BF wird, wie oben beschrieben, durch den Schlauch 12 zum $CO_2$-Sensor 1 gebracht.

Eine längere Verweildauer der Grundlinienflüssigkeit BF im gasdurchlässigen Schlauch 12 ist zulässig bzw. erwünscht, da die $CO_2$-Partialdrucke $pCO_2(BF)$ bzw. $pCO_2(Gph)$ der Grundlinienflüssigkeit BF und der umgebenden Raumluft Gph ähnliche Werte haben und sich der $CO_2$-Partialdruck $pCO_2(BF)$ der Grundlinienflüssigkeit BF bei längerer Verweildauer durch Stofftransport bzw. Gasaustausch allenfalls an den $CO_2$-Partialdruck $pCO_2(Gph)$ der Raumluft Gph angleicht und sich folglich nur geringfügig oder über eine konstante Verweildauer definiert ändert.

Aus diesen Gründen kann ein Schlauch 12 mit einem in Bezug auf eine in einer bestimmten Zeiteinheit an den $CO_2$-Sensor 1 abzugebenden Menge an Grundlinienflüssigkeit BF groß bemessenen Lumen 121 verwendet werden.

Das relativ große Lumen 121 kann vorteilhafterweise durch einen in den vom Gefäß 15 zur Kanüle 11 führenden und den Schlauch 12 enthaltenden Flüssigkeitskanal für die Grundlinienflüssigkeit BF integrierten Druckwandler 13 bestimmt werden, der die Übertragung der medizinisch bedeutsamen arteriellen Druckamplituden ermöglicht.

Anders liegen die Verhältnisse bei der vorbestimmten Eichflüssigkeit C. In der vorbestimmten Eichflüssigkeit C ist das $CO_2$ mit genau definiertem physiologischen Partialdruck $pCO_2(C)$ gelöst, der bedeutend höher als der $CO_2$-Partialdruck $pCO_2(Gph)$ der Raumluft Gph ist.

Damit aufgrund der höheren $CO_2$-Partialdruckdifferenz zwischen der vorbestimmten Eichflüssigkeit C und der Raumluft Gph keine über ein vorgebbares zulässiges Maß hinausgehende Änderung des genau definierten $CO_2$-Partialdrucks $pCO_2(C)$ dieser Flüssigkeit C entsteht, darf diese Flüssigkeit C höchstens eine durch dieses Maß bestimmte kurze Verweildauer lang im gasdurchlässigen Schlauch 16 verbleiben.

Der Schlauch 16 muß aus diesem Grund eine in Bezug auf eine in einer bestimmten Zeiteinheit an die Sensoreinrichtung 100 bzw. den $CO_2$-Sensor 1 abzugebenden Menge an vorbestimmter Eichflüssigkeit C derart klein bemessenes Lumen 161 aufweisen, daß die Eichflüssigkeit C während der Zufuhr zur Sensoreinrichtung 100 bzw. zum $CO_2$-Sensor 1 keine über das vorgebbare zulässige Maß hinausgehende Änderung der Konzentration $pCO_2(C)$ des in dieser Flüssigkeit C gelösten $CO_2$ erleidet.

Die zur Eichung des $CO_2$-Sensors 1 erforderliche zweite Eichflüssigkeit wird erfindungsgemäß durch Stofftransport bzw. Gasaustausch zwischen der vorbestimmten Eichflüssigkeit C und der Raumluft Gph, bei den vorliegenden Verhältnissen durch zeitweiliges Ausgasenlassen von $CO_2$ aus der der vorbestimmten Eichflüssigkeit C in die Raumluft Gph, erzeugt. Dazu wird die von der vorbestimmten Eichflüssigkeit C zu erzeugende Eichflüssigkeit C' der vorbestimmten anderen Konzentration $pCO_2(C')$ des $CO_2$ durch zeitweiliges Verweilenlassen der vorbestimmten Eichflüssigkeit C im Schlauch 16 und das Ausgasenlassen des $CO_2$ aus der vorbestimmten Eichflüssigkeit C durch die Schlauchwand 160 in die Raumluft Gph erzeugt.

Die Bestimmung der anderen Konzentration $pCO_2(C')$ der von der vorbestimmten Eichflüssigkeit C durch Stofftransport bzw. Gasaustausch erzeugten Eichflüssigkeit C' kann vorteilhafterweise durch eine pH-Wert-Messung erfolgen, wenn eine vorbestimmte Eichflüssigkeit C verwendet wird, bei der ein Logarithmus der Konzentration $pCO_2(C)$ des in dieser Eichflüssigkeit C gelösten $CO_2$ eine definierte, nichtkonstante Funktion f des variablen pH-Wertes $pH(C)$ dieser Eichflüssigkeit C ist. In diesem Fall kann die bestimmte andere Konzentration $pCO_2(C')$ der erzeugten Eichflüssigkeit C' durch Messen des pH-Wertes $pH(C')$ der so erzeugten Eichflüssigkeit C' mittels des über den pH-Wert der Grundlinienflüssigkeit BF und der vorbestimmten Eichflüssigkeit C bereits geeichten pH-Sensors 2 der Sensoreinrichtung 100 bestimmt werden.

Mit Hilfe des für die von der vorbestimmten Eichflüssigkeit C erzeugte Eichflüssigkeit C' vom pH-Sensor 2 ermittelten pH-Wertes $pH(C')$ kann der Logarithmus der anderen $CO_2$-Konzentration $pCO_2(C')$ dieser erzeugten Eichflüssigkeit C' durch

$$\log(pCO_2(C') = f(pH(C'))$$

bestimmt und dieser bestimmte Lagorithmus dem vom $CO_2$-Sensor 1 gemessenen Meßwert $U_{CO2}(C')$ zugeordnet werden.

Enthält jedoch die Eichflüssigkeit nur das aus Kohlensäure und $HCO_3^-$ bestehende Puffersystem und keine weiteren pH-puffernden Substanzen, ist $\log(pCO_2)$ vorteilhafterweise eine lineare Funktion des pH-Wertes pH, die gegeben ist durch

$$\log(pCO_2(C') = pH(C) - pH(C') + \log(pCO_2(C)).$$

In diesem Fall genügt zur Eichung des $CO_2$-Sensors 1 eine Zweipunkteichung, d.h. es muß zusätzlich zur genau definierten $CO_2$-Konzentration $p_{CO2}(C)$ der vorbestimmten Eichflüssigkeit nur noch eine andere $CO_2$-Konzentration $p_{CO2}(C')$ einer Eichflüssigkeit C' bestimmt werden.

Zweckmäßigerweise wird in diesem Fall eine Eichgröße $K_{pCO2}$ durch

$$-K_{pCO2} = (U_{pCO2}(C) - U_{pCO2}(C')) / (U_{pH}(C) - U_{pH}(C'))$$

gebildet.

In diesem Fall wird ein Verfahren zur Messung der Konzentration bzw. des Partialdrucks $pCO_2(B)$ von im Blut gelöstem $CO_2$ mit einem $CO_2$-Sensor 1 zum Messen der $CO_2$-Konzentration pCO2 und Erzeugen eines entsprechenden, zum Logarithmus der $CO_2$-Konzentration pCO2 proportionalen Meßwertes $U_{pCO2}$ zweckmäßigerweise so durchgeführt, daß

in beliebiger Reihenfolge nacheinander

- dem $CO_2$-Sensor 1 und pH-Sensor 2
- die vorbestimmte Eichflüssigkeit C, in der das $CO_2$ mit einem genau definierten physiologischen Partialdruck $pCO_2$ (C) und $HCO_3^-$ mit einer genau definierten physiologischen Konzentration $cHCO_3^-$ gelöst sind und die einen genau definierten physiologischen pH-Wert pH(C) aufweist, und
- die von der vorbestimmten Eichflüssigkeit C durch den Gasaustausch zwischen dieser Flüssigkeit C und der bestimmten Gasphase Gph erzeugte Eichflüssigkeit C', die sich von der vorbestimmten Eichflüssigkeit C nur durch den vom $CO_2$-Partialdruck $pCO_2(C)$ der vorbestimmten Eichflüssigkeit C verschiedenen physiologischen $CO_2$-Partialdruck $pCO_2(C')$ und den allein dadurch bedingten und vom pH-Wert pH(C) der vorbestimmten Eichflüssigkeit C verschiedenen physiologischen pH-Wert pH(C') unterscheidet, und
- dem pH-Sensor (2) überdies
- eine Grundlinienflüssigkeit BF, in der $CO_2$ mit einer einem Partialdruck von weniger als 10 Torr entsprechenden Konzentration $pCO_2(BF)$ gelöst ist und die einen definierten, vom pH-Wert pH(C) der vorbestimmten Eichflüssigkeit C verschiedenen physiologischen pH-Wert pH(BF) aufweist, zugeführt und
- der dem $CO_2$-Partialdruck $pCO_2(C)$ der vorbestimmten Eichflüssigkeit C entsprechende Meßwert $U_{pCO2}(C)$,
- der dem $CO_2$-Partialdruck $pCO_2(C')$ der von der vorbestimmten Eichflüssigkeit C erzeugten Eichflüssigkeit C' entsprechende Meßwert $U_{pCO2}(C')$,
- der dem pH-Wert pH(C) der der vorbestimmten Eichflüssigkeit C entsprechende Meßwert $U_{pH}(C)$,
- der dem pH-Wert pH(C') der von der vorbestimmten Eichflüssigkeit C erzeugten Eichflüssigkeit C' entsprechende Meßwert $U_{pH}(C')$ sowie
- der dem pH-Wert pH(BF) der Grundlinienflüssigkeit BF entsprechende Meßwert $U_{pH}(BF)$ ermittelt werden.

Mit diesen ermittelten Meßwerten $U_{pCO2}(C)$, $U_{pCO2}(C')$, $U_{pH}(C)$, $U_{pH}(C')$ und $U_{pH}(BF)$ und mit den definierten pH-Werten pH(C) und pH(BF) der vorbestimmten Eichflüssigkeit C und Grundlinienflüssigkeit BF selbst wird die Größe

$$S_{pCO2} = K_{pCO2} \cdot S_{pH}$$

mit

$$K_{pCO2} = -[(U_{pCO2}(C) - U_{pCO2}(C')) / (U_{pH}(C) - U_{pH}(C'))]$$

und

$$S_{pH} = (\Delta U_{pH}(C)) / (pH(C) - pH(BF))$$

mit

$$\Delta U_{pH}(C) = U_{pH}(C) - U_{pH}(BF)$$

gebildet, mittels welcher Größe $S_{pCO2}$

- der $CO_2$-Partialdruck $pCO_2(B)$ von im Blut B gelöstem $CO_2$ mit Hilfe
- eines durch Zuführen von Blut B zum $CO_2$-Sensor 1 ermittelten, dem $CO_2$-Partialdruck $pCO_2(B)$ des Blutes B entsprechenden Meßwertes $U_{pCO2}(B)$ und
- eines durch Zuführen von Grundlinienflüssigkeit BF zum $CO_2$-Sensor 1 ermittelten, dem $CO_2$-Partialdruck $pCO_2$ (BF) der Grundlinienflüssigkeit BF entsprechenden Meßwertes $U_{pCO2}(BF)$ vermöge

$$log(pCO2(B)) = (\Delta U_{pCO2}(B) - \Delta U_{pCO2}(C))/ S_{pCO2} + log(pCO2(C))$$

mit

$$\Delta U_{pCO2}(B) = U_{pCO2}(B) - U_{pCO2}(BF)$$

und

$$\Delta U_{pCO2}(C) = U_{pCO2}(C) - U_{pCO2}(BF)$$

bestimmbar ist.

Die vorbestimmte Eichflüssigkeit C und jede daraus erzeugte Eichflüssigkeit C′ sollte generell jeweils in allen ihren Komponenten, darunter $CO_2$, normalphysiologische Partialdruck- bzw. Konzentrationswerte und PH-Werte aufweisen.

Wie oben angedeutet, kann sich der $CO_2$-Partialdruck $pCO2(BF)$ der Grundlinienflüssigkeit BF im Schlauch 12 aufgrund unterschiedlich langer Verweildauer im Schlauch 12 und/oder des nicht konstanten atmosphärischen Luftdrucks der umgebenden Raumluft Gph laufend geringfügig ändern, d.h. der $CO_2$-Partialdruck $pCO2(BF)$ der dem $CO_2$-Sensor 1 zugeführten Grundlinienflüssigkeit BF kann driften.

In dem Fall, daß sich der $CO_2$-Partialdruck $pCO_2(BF)$ der Grundlinienflüssigkeit BF im Laufe der Zeit t ändert, werden

- die Größe $\Delta U_{pCO2}(C)$ mit einem zeitlich nahe bei der an der vorbestimmten Eichflüssigkeit C vorgenommenen $CO_2$-Messung gemessenen Meßwert $U_{pCO2}(BF(t_c))$ des $CO_2$-Partialdrucks $pCO2(BF)$ der Grundlinienflüssigkeit BF durch

$$\Delta U_{pCO2}(C) = U_{pCO2}(C) - U_{pCO2}(BF(t_c))$$

und

- die Größe $\Delta U_{pCO2}(B)$ mit einem zeitlich nahe bei der am Blut B vorgenommenen $CO_2$-Messung gemessenen Meßwert $U_{pCO2}(BF(t_b))$ des $CO_2$-Partialdrucks $pCO2(BF)$ der Grundlinienflüssigkeit BF durch

$$\Delta U_{pCO2}(B) = U_{pCO2}(B) - U_{pCO2}(BF(t_b))$$

gebildet.

Der pH-Wert pH(BF) der Grundlinienflüssigkeit sollte möglichst genau definiert sein und zeitlich konstant bleiben, so daß er nicht laufend gemessen zu werden braucht. Um dies zu ermöglichen, sollte die Grundlinienflüssigkeit pH-gepuffert sein. Die puffernden Substanzen sollten physiologisch kompatibel sein. Geeignete Stoffe sind bestimmte Phosphatverbindungen wie beispielsweise Hydrogenphosphat $HPO_4^{--}$, Dihydrogenphosphat $H_2PO_4^-$, organisches Phosphat usw..

In dem Fall jedoch, daß auch der definierte pH-Wert pH(BF) der Grundlinienflüssigkeit BF ähnlich wie deren $CO_2$-Partialdruck pCO$_2$(BF) driftet, kann so vorgegangen werden, daß

- die für die Größe $S_{pH}$ benötigten Größen $\Delta U_{pH}(C)$ und $\Delta U_{pH}(C')$ mit einem zeitlich nahe bei der an der vorbestimmten Eichflüssigkeit (C) vorgenommenen $CO_2$-Messung gemessenen Meßwert $U_{pH}(BF(tc))$ des pH-Werts $pH(BF(t_c))$ der Grundlinienflüssigkeit (BF) durch

$$\Delta U_{pH}(C) = U_{pH}(C) - U_{pH}(BF(t_C))$$

$$\Delta U_{pH}(C') = U_{pH}(C) - U_{pH}(BF(t_C))$$

gebildet werden.

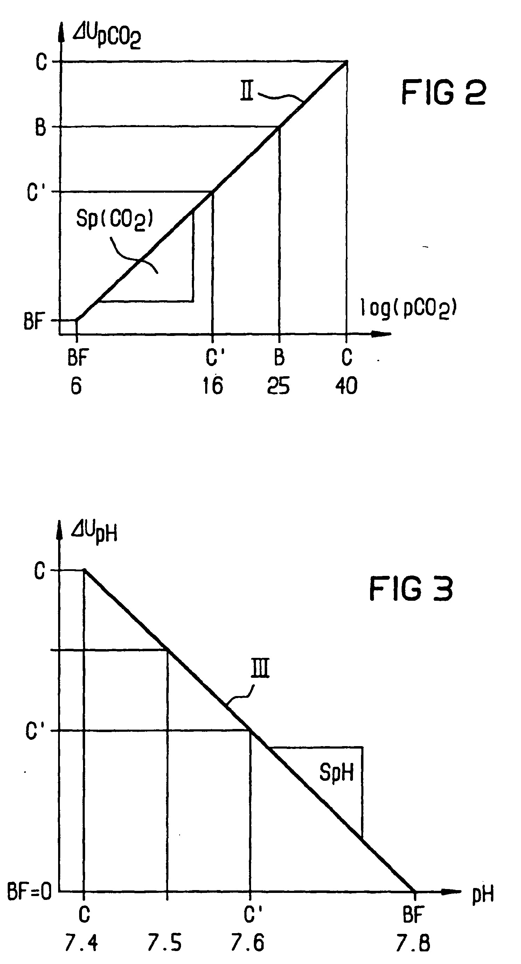

In der Figur 2 ist in einem Diagramm beispielhaft eine Charakteristik eines $CO_2$-Sensors 1 zum Messen der Konzentration bzw. des Partialdrucks pCO$_2$ von in einer Flüssigkeit gelöstem $CO_2$ dargestellt. In diesem Diagramm ist der auf den $CO_2$-Meßwert $U_{pCO2}(BF)$ einer Grundlinienflüssigkeit bezogene $CO_2$-Messwert des Sensors 1, d.h. die Größe oder Differenz $\Delta U_{pCO2}$, in Abhängigkeit von log(pCO$_2$) für einen beispielhaften Bereich von log(pCO$_2$) dargestellt, der einem $CO_2$-Partialdruckbereich von 0 bis etwas über 40 Torr entspricht. Der Zusammenhang zwischen $\Delta U_{pCO2}$ und log(pCO$_2$) ist durch die Gerade II mit der Steigung $S_{pCO2}$ gegeben.

Beispielsweise ist in der Figur 2 angenommen, daß pCO$_2$(BF) = 6 Torr, pCO$_2$(B) = 25 Torr, pCO$_2$(C) = 40 Torr und pCO$_2$(C') = 16 Torr betragen.

Die Figur 3 zeigt in einem Diagramm beispielhaft eine Charakteristik eines pH-Sensors 2 zum Messen des pH-Wertes einer Flüssigkeit. In diesem Diagramm ist der auf den pH-Meßwert $U_{pH}(BF)$ einer Grundlinienflüssigkeit bezogene pH-Messwert des Sensors 2, d.h. die Größe oder Differenz $\Delta U_{pH}$, in Abhängigkeit vom pH-Wert für einen beispielhaften pH-Bereich von 7,0 bis 7,8. Der Zusmmenhang zwischen $\Delta U_{pH}$ und dem pH-Wert ist durch die Gerade III mit der Steigung $S_{pH}$ gegeben.

In der Figur 2 ist beispielsweise pH(BF) = 7,8, pH(B) = 7,5, pH(C) = 7,4 und pH(C') = 7,6 angenommen.

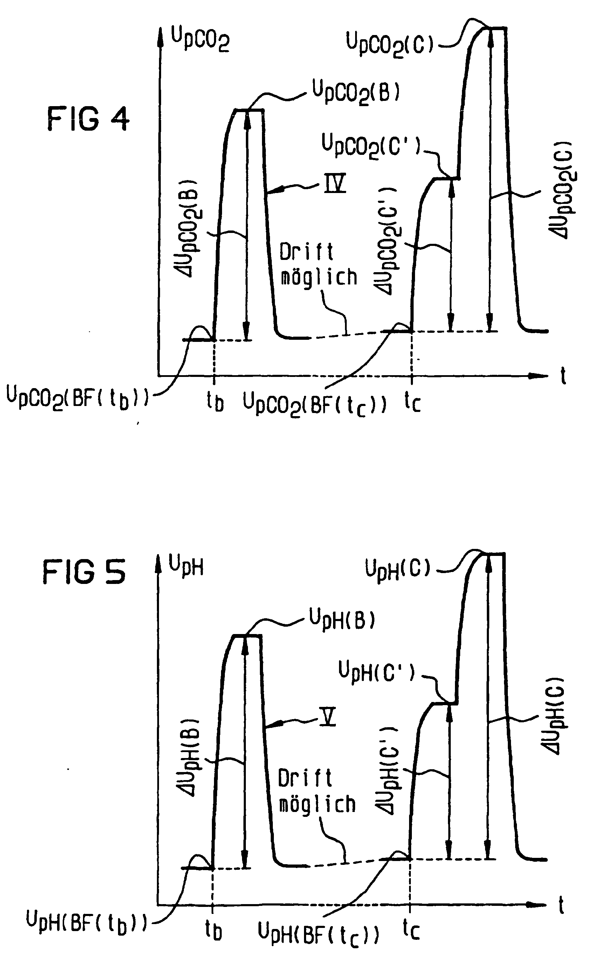

Die Figur 4 zeigt eine bei dem oben beschriebenen erfindungsgemäßen Verfahren zu Messung der $CO_2$-Konzentration von Blut B mit dem $CO_2$-Sensor 1 aufgenommene Messkurve IV, die den Verlauf des von diesem Sensor 1 gelieferten Meßwerts Up$_{CO2}$ über der Zeit t darstellt.

Die Figur 5 zeigt eine bei diesem Verfahren mit dem pH-Sensor 2 aufgenommene Messkurve V, die den Verlauf des von diesem Sensor 2 gelieferten Meßwerts $U_{pH}$ über der Zeit t darstellt. In dieser Figur bedeutet $\Delta U_{pH}(B)$ die Differenz $U_{pH}(B) - U_{pH}(BF)$.

Die aus den Figuren 2 bis 5 entnehmbaren Verhältnisse liegen im wesentlichen dem folgenden konkreten Ausführungsbeispiel des erfindungsgemäßen dieses Verfahrens zu Messung der $CO_2$-Konzentration von Blut B zugrunde.

Bei diesem konkreten Ausführungsbeispiel wurden unter Normalbedingung:

eine pH- gepufferte Grundlinienflüssigkeit BF in Form einer luftgesättigten wässerigen Lösung, die bei 37°C einen genau definierten physiologischen pH-Wert pH(BF) von beispielsweise 7,8,
$O_2$ mit einem physiologischen Partialdruck pO$_2$(BF) von etwa 185 Torr,
$CO_2$ mit einem ungenau bestimmten Partialdruck pCO$_2$ (BF) von weniger als 10 Torr,
$K^+$ in einer genau definierten Konzenration cK$^+$(BF) von beispielsweise 2,0 mmol/l,
$Ca^{2+}$ in einer genau definierten Konzenration cCa$^{2+}$(BF) von beispielsweise 1,0 mmol/l,
Lactat in einer genau definierten Konzenration cL(BF) von beispielsweise 0,0 mmol/l aufwies
und
- eine vorbestimmte Eichflüssigkeit C in Form einer wässerigen Lösung, die bei 37°C
einen genau definierten physiologischen pH-Wert pH(BF) von beispielsweise 7,40,
$O_2$ mit einem genau definierten physiologischen Partialdruck pO$_2$(C) von beispielsweise 185 Torr,
$CO_2$ mit einem genau definierten physiologischen Partialdruck pCO$_2$(C) von beispielsweise 40 Torr,
$K^+$ in einer genau definierten Konzenration cK$^+$(BF) von beispielsweise 4,0 mmol/l,

Ca$^{2+}$ in einer genau definierten Konzenration cCa$^{2+}$(BF) von beispielsweise 2,0 mmol/l,
Lactat in einer genau definierten Konzenration cL(BF) von beispielsweise 5,0 mmol/l aufwies,

verwendet.

Eine Messung am Blut B wurde etwa alle 2 bis 3 Minuten durchgeführt. Jede dieser Messungen umfaßt zumindest die Gewinnung von $U_{pCO2}$(B), $U_{pCO2}$(BF) kann aber auch die Gewinnung von $U_{pH}$(B) und $U_{pH}$(BF) und/oder eines oder mehrerer der im Blut B und der Grundlinienflüssigkeit BF enthaltenen anderen obigen Stoffe sowohl für das Blut B als auch die Grundlinienflüssigkeit BF beinhalten.

$U_{pH}$(BF) muß nur einmal ermittelt werden, da die Grundlinienflüssigkeit BF gepuffert und pH(BF) zeitlich konstant ist. Andernfalls muß auch $U_{pH}$(BF) je nach Drift mehr oder weniger oft ermittelt werden.

$U_{pCO2}$(BF) sollte jeweils zu einem nahe bei einer am Blut B vorgenommenen Messung liegenden Zeitpunkt $t_b$ gewonnen werden. Das gleiche gilt, wenn $U_{pH}$(BF) zu ermitteln ist und pH(BF) driftet.

Eine Messung an der vorbestimmten Eichflüssigkeit C zu einer Eichung der Sensoren wurde etwa alle 1 bis 3 Stunden durchgeführt. Jede dieser Messungen umfaßt zumindest die Gewinnung von $U_{pCO2}$(C), $U_{pCO2}$(BF), $U_{pH}$(C), kann aber auch die Gewinnung von Meßwerten eines oder mehrerer der in der vorbestimmten Eichflüssigkeit C und der Grundlinienflüssigkeit BF enthaltenen anderen obigen Stoffe sowohl für die vorbestimmte Eichflüssigkeit C als auch die Grundlinienflüssigkeit BF beinhalten. Außerdem muß spätestens jetzt $U_{pH}$(BF) gewonnen werden, da dieser Meßwert zur Eichung des $CO_2$-Sensors benötigt wird.

$U_{pCO2}$(BF) sollte bei der Eichung zu einem nahe bei einer an der vorbestimmten Eichflüssigkeit C vorgenommenen Messung liegenden Zeitpunkt $t_c$ gewonnen werden. Das gleiche gilt für $U_{pH}$(BF), wenn pH(BF) driftet.

Die Grundlinienflüssigkeit BF wurde den Sensoren 1 und 2 durch einen beispielsweise 1 bis 2 m langen Schlauch 12 mit einem Lumen 121 von beispielsweise 1,5 mm zugeführt. Die Verweildauer der Grundlinienflüssigkeit BF in diesem Schlauch 12 betrug dabei beispielsweise 30 min.

Die vorbestimmte Eichflüssigkeit C wurde den Sensoren 1 und 2 durch einen beispielsweise 1 bis 2 m langen Schlauch 16 mit einem Lumen 161 von beispielsweise 0,5 mm zugeführt. Die Verweildauer der Eichflüssigkeit C in diesem Schlauch 16 betrug dabei einige Sekunden.

$U_{pCO2}$(C′) und $U_{pH}$(C′) werden jeweils zeitlich kurz vor der Messung an der vorbestimmten Eichflüssigkeit C dadurch gewonnen, daß die seit der letzten Messung an der vorbestimmten Eichflüssigkeit C längere Zeit im Schlauch 16 verbliebene Eichflüssigkeit, aus der $CO_2$ ausgegast ist und welche die aus der vorbestimmten Eichflüssigkeit C gewonnene Eichflüssigkeit C′ der anderen Konzentration $pCO_2$(C′) bildet, sowohl dem CO2-Sensor 1 als auch dem pH-Sensor 2 zugeführt wird.

Danach sind alle zur genauen Ermittlung von log($pCO_2$(B)) erforderlichen Meßwerte gewonnen und pCO2(B) kann bestimmt werden, da $pCO_2$(C) genau bekannt ist.

## Patentansprüche

1. Verfahren zur Eichung eines Sensors (1) zur Messung der Konzentration (pG) eines in einer Flüssigkeit (B, C, C′) gelösten Gases (G) und Erzeugen eines der gemessenen Konzentration (pG) entsprechenden Meßwertes ($U_{pG}$; $I_{pG}$), wobei dem Sensor (1) zur Eichung

   - zuzmindest zwei jeweils das Gas (G) in genau definierter aber voneinander verschiedener Konzentration (pG (C), pG(C')) gelöst enthaltende Eichflüssigkeiten (C, C′) durch einen Schlauch (16) zugeführt werden und der Schlauch (16) eine gasdurchlässige Schlauchwand (160) hat und
   - die diesen verschiedenen Konzentrationen (pG(C), pG(C')) entsprechenden Meßwerte ($U_{pG}$(C), $U_{pG}$(C′); $I_{pG}$(C), $I_{pG}$(C′)) ermittelt werden,

   **dadurch gekennzeichnet,**
   **daß** zumindest eine der beiden Eichflüssigkeinten von einer vorbestimmten Eichflüssigkeit durch eine Änderung der Konzentration (pG(C)) des in dieser vorbestimmten Eichflüssigkeit (C) gelösten Gases (G) auf eine vorbestimmte andere Konzentration (pG(C′)) dieses Gases (G) erzeugt wird,
   und **daß** die Änderung der Konzentration (pG(C)) des in dieser vorbestimmten Eichflüssigkeit (C) gelösten Gases (G) auf die vorbestimmte andere Konzentration (pG(C′)) durch einen Stofftransport durch die gasdurchlässige Schlauchwand (160) zwischen der vorbestimmten Eichflüssigkeit (C) und einer vorbestimmten Gasphase (Gph) erzeugt wird.

2. verfahren nach Anspruch 1,
   **dadurch gekennzeichnet,**

- **daß** eine Gasphase (Gph) verwendet wird, die das Gas (G) mit einem vom Partialdruck (pG(C)) des in der vorbestimmten Eichflüssigkeit (C) gelösten Gases (G) verschiedenen Partialdruck (pG(Gph)) enthält, so daß zwischen der Gasphase (Gph) und der vorbestimmten Eichflüssigkeit (C) ein Partialdruckunterschied ($\Delta$pG (Gph,G)) bezüglich des Gases (G) besteht, und

- **daß** ein durch diesen Partialdruckunterschied ($\Delta$pG(A,G)) bedingter Vorgang eines Ausgleichs dieses Druckunterschieds ($\Delta$pG(A,G)) solange aufrecherhalten wird, bis eine Eichflüssigkeit (C´) mit einem der vorbestimmten anderen Konzentration entsprechenden Partialdruck (pG(C´)) dieses Gases (G) vorliegt.

3. Verfahren nach Anspruch 1 oder 2,
   **dadurch gekennzeichnet,**
   **daß** eine aus Luft bestehende Gasphase (Gph) verwendet wird.

4. Verfahren nach einem der Ansprüche 1 bis 3,
   **dadurch gekennzeichnet,**

   - **daß** die vorbestimmte Eichflüssigkeit (C) dem Sensor (1) durch einen Schlauch (16) mit einer von der Gasphase (Gph) umgebenen gasdurchlässigen Schlauchwand (160) und einem in bezug auf eine in einer bestimmten Zeiteinheit an den Sensor

     (1) abzugebenden Menge an vorbestimmter Eichflüssigkeit (C) derart klein bemessenen Innenquerschnitt (161) zugeführt wird, daß eine Eichflüssigkeit (C, C´) während der Zufuhr zum Sensor (1) keine über ein vorgebbares zulässiges Maß hinausgehende Änderung der Konzentration (pG(C)) des in dieser Flüssigkeit (C) gelösten Gases (G) aufgrund eines Stofftransportes zwischen dieser Flüssigkeit (C) und der Gasphase (Gph) durch die Schlauchwand (160) erleidet und

   - **daß** die von der vorbestimmten Eichflüssigkeit (C) zu erzeugende Eichflüssigkeit (C´) der vorbestimmten anderen Konzentration (pG(C´)) durch zeitweiliges Verweilenlassen der vorbestimmten Eichflüssigkeit (C) im Schlauch (3) und einen Stofftransport zwischen dieser Flüssigkeit (C) und der Gasphase (Gph) durch die Schlauchwand (3) erzeugt wird.

5. Verfahren nach Anspruch 4,
   **dadurch gekennzeichnet,**
   **daß** die vorbestimmte Eichflüssigkeit (C) einem gasundurchlässigen Gefäß (18) entnommen wird.

6. Verfahren nach einem der vorhergehenden Ansprüche,
   **dadurch gekennzeichnet,**
   **daß** der Sensor (1) einen der Konzentration (pG) des in der dem Sensor (1) zugeführten Flüssigkeit (B, C, C´) gelösten Gases (G) direkt proportionalen Meßwert ($I_{kG}$(B), $I_{kG}$(C), $I_{kG}$(C')) erzeugt.

7. Verfahren nach einem der Ansprüche 1 bis 5,
   **dadurch gekennzeichnet,**
   **daß** der Sensor (1) einen zu einem Logarithmus der Konzentration (pG) des in der dem Sensor (1) zugeführten Flüssigkeit (B, C, C´) gelösten Gases (G) proportionalen Meßwert ($U_{kG}$(B), $U_{kG}$(C), $U_{kG}$(C´)) erzeugt.

8. Verfahren nach einem der vorhergehenden Ansprüche, insbesondere nach Anspruch 7,
   **dadurch gekennzeichnet,**

   - **daß** eine vorbestimmte Eichflüssigkeit (C) verwendet wird, bei welcher die Konzentration (pG(C)) des in dieser Eichflüssigkeit (C) gelösten Gases (G) eine definierte, nichtkonstante Funktion (f) des variablen pH-Werts (pH (C)) dieser Eichflüssigkeit (C) ist, und
   - **daß** die vorbestimmte andere Konzentration (pG(C´)) der von der vorbestimmten Eichflüssigkeit (C) erzeugten Eichflüssigkeit (C´) über den pH-Wert (pH(C´) der so erzeugten Eichflüssigkeit (C´) genau bestimmt wird.

9. Verfahren nach Anspruch 8,
   **dadurch gekennzeichnet,**
   **daß** eine vorbestimmte Eichflüssigkeit (C) verwendet wird, bei welcher ein Logarithmus der Konzentration (pG (C)) des in dieser Eichflüssigkeit (C) gelösten Gases (G) eine nichtkonstante lineare Funktion des variablen pH-Werts (pH) dieser Eichflüssigkeit (C) ist

**10.** Verfahren nach Anspruch 7 und Anspruch 9,
**dadurch gekennzeichnet,**

- **daß** die vorbestimmte Eichflüssigkeit (C) und die daraus erzeugte Eichflüssigkeit (C′) der vorbestimmten anderen Konzentration (pG(C′)) einem pH-Sensor (2) zum Messen des pH-Wertes und Erzeugen eines zum pH-Wert (pH) proportionalen Meßwerts ($U_{pH}$) zugeführt und die den voneinander verschiedenen pH-Werten dieser beiden Eichflüssigkeiten (C, C′) entsprechenden Meßwerte ($U_{pH}(C)$, $U_{pH}(C')$)
ermittelt werden, und
- **daß** mit den vom einen Sensor (1) ermittelten Meßwerten ($U_{pG}(C)$, $U_{pG}(C')$) und den vom pH-Sensor (2) ermittelten Meßwerten ($U_{pH}(C)$, $U_{pH}(C')$) die Größe

$$-K_{pG} = (U_{pG}(C) - U_{pG}(C'))/(U_{pH}(C) - U_{pH}(C'))$$

gebildet wird.

**11.** Verfahren nach einem der vorhergehenden Ansprüche,
**dadurch gekennzeichnet,**
**daß** als Sensor (1) zur Messung der Konzentration eines in einer Flüssigkeit (B, C, C′) gelösten Gases (G) ein $CO_2$-Sensor (1) zur Messung des Partialdrucks ($pCO_2$) von in einer Flüssigkeit (B) gelöstem $CO_2$ verwendet wird

**12.** Verfahren nach einem der vorhergehenden Ansprüche,
**dadurch gekennzeichnet,**
**daß** als Sensor (1) zur Messung der Konzentration eines in einer Flüssigkeit (B, C, C′) gelösten Gases (G) ein Sensor zur Messung des Partialdrucks (pG) von in einer physiologischen Körperflüssigkeit (B) und/oder einem physiologischen Gewebe gelöstem Gas (G) verwendet wird.

**13.** Verfahren nach Anspruch 11 und 12,
**dadurch gekennzeichnet,**
**daß** ein $CO_2$-Sensor (1) zur Messung der des Partialdrucks ($pCO_2$) von in Blut (B) gelöstem $CO_2$ verwendet wird.

**14.** Verfahren nach Anspruch 9 und 11 oder 13,
**dadurch gekennzeichnet,**
**daß** eine vorbestimmte Eichflüssigkeit (C) verwendet wird, in der $HCO_3^-$ gelöst ist.

**15.** Verfahren zur Messung des Partialdrucks ($pCO_2$ (B)) von in Blut (B) gelöstem $CO_2$ mit einem $CO_2$-Sensor (1) zum Messen des $CO_2$-Partialdrucks ($pCO_2$) und Erzeugen eines entsprechenden, zum Logarithmus des $CO_2$-Partial-drucks ($pCO_2$) proportionalen Meßwerts ($U_{pCO2}$) unter Verwendung eines Verfahrens nach Anspruch 10 und 13 und 14.
**dadurch gekennzeichnet, daß**
in beliebiger Reihenfolge nacheinander

- dem $CO_2$-Sensor (1) und pH-Sensor (2)
- die vorbestimmte Eichflüssigkeit (C), in der das $CO_2$ mit einem genau definierten physiologischen Partialdruck ($pCO_2$(C)) und $HCO_3^-$ mit einer genau definierten physiologischen Konzentration ($cHCO_3^-$) gelöst sind und die einen genau definierten physiologischen pH-Wert (pH(C)) aufweist, und
- die von der vorbestimmten Eichflüssigkeit (C) durch Stofftransport zwischen dieser Flüssigkeit (C) und der vorbestimmten Gasphase (Gph) erzeugte Eichflüssigkeit (C′), die sich von der vorbestimmten Eichflüssigkeit (C) nur durch den vom $CO_2$-Partialdruck ($pCO_2$(C)) der vorbestimmten Eichflüssigkeit (C) verschiedenen phy-siologischen $CO_2$-Partialdruck ($pCO_2$(C′)) und den allein **dadurch** bedingten und vom pH-Wert (pH(C)) der vorbestimmten Eichflüssigkeit (C) verschiedenen physiologischen pH-Wert (pH(C′)) unterscheidet, und
- dem pH-Sensor (2) überdies
- eine Grundlinienflüssigkeit (BF), in der $CO_2$ mit einem Partialdruck ($pCO_2$(BF)) von weniger als 10 Torr gelöst ist und die einen definierten, vom pH-Wert (pH(C)) der vorbestimmten Eichflüssigkeit (C) verschiedenen physiologischen pH-Wert (pH(BF)) aufweist,

zugeführt und

- der dem $CO_2$-Partialdruck ($pCO_2(C)$) der vorbestimmten Eichflüssigkeit (C) entsprechende Meßwert ($U_{pCO2}$(C)),
- der dem $CO_2$-Partialdruck ($pCO_2(C')$) der von der vorbestimmten Eichflüssigkeit (C) erzeugten Eichflüssigkeit (C') entsprechende Meßwert ($U_{pCO2}(C')$),
- der dem pH-Wert (pH(C)) der der vorbestimmten Eichflüssigkeit (C) entsprechende Meßwert ($U_{pH}$(C),
- der dem pH-Wert (pH(C')) der von der vorbestimmten Eichflüssigkeit (C) erzeugten Eichflüssigkeit (C') entsprechende Meßwert ($U_{pH}$(C') sowie
- der dem pH-Wert (pH(BF)) der Grundlinienflüssigkeit (BF) entsprechende Meßwert ($U_{pH}$(BF))

ermittelt werden und

- mit diesen Meßwerten ($U_{pCO2}$(C), $U_{pCO2}$(C'), $U_{pH}$(C), $U_{pH}$(C'), $U_{pH}$(BF)) und den definierten pH-Werten (pH(C), pH(BF)) der vorbestimmten Eichflüssigkeit (C) und Grundlinienflüssigkeit (BF) selbst die Größe

$$S_{pCO2} = K_{pCO2} \cdot S_{pH}$$

mit

$$K_{pCO2} = -[(U_{pCO2}(C) - U_{pCO2}(C'))/(U_{pH}(C) - U_{pH}(C'))]$$

und

$$S_{pH} = (\Delta U_{pH}(C))/(pH(C) - pH(BF))$$

mit

$$\Delta U_{pH}(C) = U_{pH}(C) - U_{pH}(BF)$$

gebildet wird, mittels welcher Größe $S_{pCO2}$
- der $CO_2$-Partialdruck ($pCO_2(B)$) von im Blut (B) gelöstem $CO_2$ mit Hilfe
- eines durch Zuführen von Blut (B) zum $CO_2$-Sensor (1) ermittelten, dem $CO_2$-Partialdruck ($pCO_2(B)$) des Blutes (B) entsprechenden Meßwertes ($U_{pCO2}$(B)) und
- eines durch Zuführen von Grundlinienflüssigkeit (BF) zum $CO_2$-Sensor (1) ermittelten, dem $CO_2$-Partialdruck ($pCO_2(BF)$) der Grundlinienflüssigkeit (BF) entsprechenden Meßwertes ($U_{pCO2}$(BF))

vermöge
bestimmbar ist, wobei

$$\Delta U_{pCO2}(B) = U_{pCO2}(B) - U_{pCO2}(BF),$$

$$\Delta U_{pCO2}(C) = U_{pCO2}(C) - U_{pCO2}(BF)$$

ist.

**16.** Verfahren nach Anspruch 15,

**dadurch gekennzeichnet,**
**daß** sich der $CO_2$-Partialdruck (pCO2(BF)) der Grundlinienflüssigkeic (BF) im Laufe der Zeit (t) ändern, und daß

- die Größe $\Delta U_{pCO2}$(C) mit einem zeitlich nahe bei der an der vorbestimmten Eichflüssigkeit (C) vorgenommenen $CO_2$-Messung gemessenen Meßwert $U_{pCO2}(BF(t_c))$ des $CO_2$-Partialdrucks (pCO2(BF)) der Grundlinienflüssigkeit (BF) durch

$$\Delta U_{pCO2}(C) = U_{pCO2}(C) - U_{pCO2}(BF(t_c))$$

und
- die Größe $\Delta U_{pCO2}$(B) mit einem zeitlich nahe bei der am Blut (B) vorgenommenen $CO_2$-Messung gemessenen Meßwert
$U_{pCO2}(BF(t_b))$ des $CO_2$-Partialdrucks (pCO2(BF)) der Grundlinienflüssigkeit (BF) durch

$$\Delta U_{pCO2}(B) = U_{pCO2}(B) - U_{pCO2}(BF(t_b))$$

gebildet werden.

17. Verfahren nach Anspruch 15 oder 16,
**dadurch gekennzeichnet,**
**daß** eine vorbestimmte Eichflüssigkeit (C) verwendet wird, in der $NaHCO_3$ in einer physiologischen Konzentration gelöst ist.

18. verfahren nach einem der Ansprüche 15 bis 17,
**dadurch gekennzeichnet,**
**daß** die von der vorbestimmte Eichflüssigkeit (C) erzeugte Eichflüssigkeit (C′) durch zeitweiliges Ausgasenlassen von $CO_2$ aus der vorbestimmte Eichflüssigkeit (C) erzeugt wird.

19. Verfahren nach Anspruch 18,
**dadurch gekennzeichnet,**

- **daß** die vorbestimmte Eichflüssigkeit (C) den Sensoren (1, 2) durch einen Schlauch (16) mit einer von der Gasphase (Gph) umgebenen gasdurchlässigen Schlauchwand (160) und einem in bezug auf eine in einer bestimmten Zeiteinheit an die Sensoren (1, 2) abzugebenden Menge an vorbestimmter Eichflüssigkeit (C) derart klein bemessenen Innenquerschnitt (161) zugeführt wird, daß eine Eichflüssigkeit (C, C′) während der Zufuhr zu den Sensoren (1, 2) keine über ein vorgebbares zulässiges Maß hinausgehende Änderung der Konzentration ($pCO_2$(C)) des in dieser Flüssigkeit (C) gelösten $CO_2$ erleidet und
- **daß** die von der vorbestimmten Eichflüssigkeit (C) zu erzeugende Eichflüssigkeit (C′) der vorbestimmten anderen Konzentration ($pCO_2$(C′)) des $CO_2$ durch zeitweiliges Verweilenlassen der vorbestimmten Eichflüssigkeit (C) im Schlauch (16) und einen Stofftransport zwischen dieser Flüssigkeit (C) und der Gasphase (Gph) durch die Schlauchwand (160) erzeugt wird.

20. verfahren nach einem der Ansprüche 15 bis 19,
**dadurch gekennzeichnet,**
**daß** eine unter Normalbedingungen luftgesättigte Grundlinienflüssigkeit (BF) verwendet wird.

21. Verfahren nach einem der Ansprüche 16 bis 20,
**dadurch gekennzeichnet,**
**daß** die Grundlinienflüssigkeit (BF) den Sensoren (1, 2) durch einen gasdurchlässigen Schlauch (12) mit einem in bezug auf eine in einer bestimmten Zeiteinheit an die Sensoren (1, 2) abzugebenden Menge an Grundlinienflüssigkeit (BF) derart groß bemessenen Innenquerschnitt (121) zugeführt wird, daß die Grundlinienflüssigkeit (BF) während der Zufuhr zu den Sensoren (1, 2) eine für eine Anpassung der Grundlinienflüssigkeit (BF) an einen Umgebungsdruck ausreichende Verweildauer lang im Schlauch (12) verbleibt.

**22.** Verfahren nach einem der Ansprüche 15 bis 21,
**dadurch gekennzeichnet,**
**daß** die Grundlinienflüssigkeit (BF) einem gasundurchlässigen Gefäß (15) entnommen wird.

**23.** Verfahren nach einem der Ansprüche 15 bis 22,
**dadurch gekennzeichnet,**
**daß** eine einmal ermittelte definierte Größe $S_{pCO2}$ zur mehrfachen Bestimmung des der $CO_2$-Partialdrucks ($pCO_2$ (B)) von Blut (B) durch jeweilige Ermittlung des dem $CO_2$-Partialdruck ($pCO_2$(B)) des Blutes (B) entsprechenden Meßwertes ($U_{pCO2}$(B)) und des dem $CO_2$-Partialdruck ($pCO_2$(BF)) der Grundlinienflüssigkeit (BF) entsprechenden Meßwertes ($U_{pCO2}$(BF)) verwendet wird.

**Claims**

**1.** A method for calibrating a sensor (1) for measuring a concentration (pG) of a gas (G) dissolved in a fluid (B, C, C') and for generating a measured value ($U_{pG}$; $I_{pG}$) corresponding to the measured concentration (pG), the sensor (1) being supplied for calibration with:

- at least two calibration fluids (C, C') through a tube (16) and the tube (16) has a gas-permeable tube wall (160) and, each of which calibration fluids contains the gas (G) in a precisely defined, but different dissolved concentration (pG(C), pG(C'')) and
- the measurement values ($U_{pG}$(C), $U_{pG}$(C') ; $I_{pG}$(C), $I_{pG}$(C')) corresponding to these different concentrations (pG(C), pG(C')) are determined,

**characterised**
**in that** at least one of the two calibration fluids is produced from a predetermined calibration fluid by changing the concentration (pG(C)) of the gas (G) dissolved in this predetermined calibration fluid (C) to a predetermined, other concentration (pG(C')) of this gas (G) and
**in that** the change in concentration (pG(C)) of the gas (G) dissolved in this predetermined calibration fluid (C) to the predetermined, other concentration (pG(C')) is produced by material transport through the gas-permeable tube wall (160) between the predetermined calibration fluid (C) and a predetermined gas phase (Gph).

**2.** A method according to claim 1,
**characterised in that**
a gas phase (Gph) is used which contains the gas (G) with a partial pressure (pG(Gph)) which differs from the partial pressure (pG(C)) of the gas (G) dissolved in the predetermined calibration fluid (C), such that there is a partial pressure difference ($\Delta pG(Gph,G)$) with regard to the gas (G) between the gas phase (Gph) and the predetermined calibration fluid (C), and
**in that** a process of equalisation of this pressure difference ($\Delta pG(A,G)$), driven by this partial pressure difference ($\Delta pG(A,G)$), is maintained until a calibration fluid (C') having a partial pressure (pG(C')) of this gas (G) which corresponds to the predetermined, other concentration is obtained.

**3.** A method according to claim 1 or claim 2,
**characterised in that**
a gas phase (Gph) consisting of air is used.

**4.** A method according to any one of claims 1 to 3,
**characterised**
**in that** the predetermined calibration fluid (C) is supplied to the sensor (1) through a tube (16) having a gas-permeable tube wall (160) surrounded by the gas phase (Gph) and an internal cross-section (161) which, relative to a quantity of predetermined calibration fluid (C) to be provided in a determined unit of time to the sensor (1), is of a sufficiently small size that, while being supplied to the sensor (1), a calibration fluid (C, C') does not undergo a change going beyond a specifiable, allowed extent in the concentration (pG(C)) of the gas (G) dissolved in this fluid (C) due to material transport between this fluid (C) and the gas phase (Gph) through the tube wall (160) and,
**in that** the calibration fluid (C') of the predetermined other concentration (pG(C')) which is to be produced from the predetermined calibration
fluid (C) is produced by allowing the predetermined calibration fluid (C) to dwell temporarily in the tube (3) and material transport between this fluid (C) and the gas phase (Gph) through the tube wall (3).

**5.** A method according to claim 4,
**characterised in that**
the predetermined calibration fluid (C) is taken from a gas-impermeable vessel (18).

**6.** A method according to any one of the preceding claims,
**characterised in that**
the sensor (1) generates a measured value ($I_{kG}(B)$, $I_{kG}(C)$, $I_{kG}(C')$) which is directly proportional to the concentration (pG) of the gas (G) dissolved in the fluid (B, C, C') supplied to the sensor (1).

**7.** A method according to any one of claims 1 to 5,
**characterised in that**
the sensor (1) generates a measured value ($U_{kG}(B)$, $U_{kG}(C)$, $U_{kG}(C')$) which is proportional to a logarithm of the concentration (pG) of the gas (G) dissolved in the fluid (B, C, C') supplied to the sensor (1).

**8.** A method according to any one of the preceding claims, in particular according to claim 7,
**characterised in that**
a predetermined calibration fluid (C) is used in which the concentration (pG(C)) of the gas (G) dissolved in this calibration fluid (C) is a defined, non-constant function (f) of the variable pH value (pH(C)) of this calibration fluid, and **in that** the predetermined, other concentration (pG(C')) of the calibration fluid (C') produced from the predetermined calibration fluid (C) is precisely determined via the pH value (pH(C')) of the calibration fluid (C') produced in this manner.

**9.** A method according to claim 8,
**characterised in that**
a predetermined calibration fluid (C) is used in which a logarithm of the concentration (pG(C)) of the gas (G) dissolved in this calibration fluid (C) is a non-constant, linear function of the variable pH value (pH) of this calibration fluid (C).

**10.** A method according to claim 7 and claim 9,
**characterised**
**in that** the predetermined calibration fluid (C) and the calibration fluid (C') of the predetermined, other concentration (pG(C')) produced therefrom is supplied to a pH sensor (2) for measuring the pH value and generating a measured value ($U_{pH}$) proportional to the pH value (pH) and the measured value ($U_{pH}(C)$, $U_{pH}(C')$) corresponding to the differing pH values of these two calibration fluids (C, C') are determined and **in that** the variable

$$-K_{pG} = (U_{pG}(C) - U_{pG}(C'))/(U_{pH}(C) - U_{pH}(C'))$$

is formed from the measured values ($U_{pG}(C)$, $U_{pG}(C')$) determined by a sensor (1) and the measured values ($U_{pH}(C)$, $U_{pH}(C')$) determined by the pH sensor (2).

**11.** A method according to any one of the preceding claims,
**characterised in that**
a $CO_2$ sensor (1) for measuring the partial pressure ($pCO_2$) of $CO_2$ dissolved in a fluid (B) is used as the sensor (1) for measuring the concentration of a gas (G) dissolved in a fluid (B, C, C').

**12.** A method according to any one of the preceding claims,
**characterised in that**
a sensor for measuring the partial pressure (pG) of a gas (G) dissolved in a physiological body fluid (B) and/or in a physiological tissue is used as the sensor (1) for measuring the concentration of a gas (G) dissolved in a fluid (B, C, C').

**13.** A method according to claim 11 and claim 12,
**characterised in that**
a $CO_2$ sensor (1) is used for measuring the partial pressure ($pCO_2$) of $CO_2$ dissolved in blood (B) .

**14.** A method according to claim 9 and 11 or 13,

**characterised in that**
a predetermined calibration fluid (C) is used in which $HCO_3^-$ is dissolved.

**15.** A method for measuring the partial pressure ($pCO_2(B)$) of $CO_2$ dissolved in blood (B) with a $CO_2$ sensor (1) for measuring the $CO_2$ partial pressure ($pCO_2$) and generating a measured value ($U_{pCO2}$) proportional to the logarithm of $CO_2$ partial pressure ($pCO_2$) using a method according to claim 10 and 13 and 14,
**characterised in that**
successively, in an arbitrary sequence,

- the $CO_2$ sensor (1) and pH sensor (2) are supplied with
- the predetermined calibration fluid (C) in which the $CO_2$ is dissolved with a precisely defined physiological partial pressure $pCO_2(C)$ and in which $HCO_3^-$ is dissolved with a precisely defined physiological concentration ($cHCO_3^-$) and which has a precisely defined physiological pH value (pH(C)), and
- the calibration fluid (C') produced from the predetermined calibration fluid (C) by material transport between this fluid (C) and the predetermined gas phase (Gph), this calibration fluid (C') differing from the predetermined calibration fluid (C) only on the basis of the physiological $CO_2$ partial pressure ($pCO_2(C')$), which differs from the $CO_2$ partial pressure ($pCO_2(C)$) of the predetermined calibration fluid (C), and on the basis of the physiological pH value (pH(C')) determined solely thereby and which differs from the pH value (pH(C)) of the predetermined calibration fluid (C), and
- the pH sensor (2) is also supplied with
- a baseline fluid (BF) in which $CO_2$ is dissolved with a partial pressure ($pCO_2(BF)$) of less than 10 Torr and that has a defined physiological pH value (pH(BF)) differing from the pH value (pH(C)) of the predetermined calibration fluid (C), and
- the measured value ($U_{pCO2}(C)$) corresponding to the $CO_2$ partial pressure ($pCO_2(C)$) of the predetermined calibration fluid (C),
- the measured value ($U_{pCO2}(C')$) corresponding to the $CO_2$ partial pressure ($pCO_2(C')$) of the calibration fluid (C') produced from the predetermined calibration fluid (C),
- the measured value ($U_{pH}(C)$) corresponding to the pH value (pH(C)) of the predetermined calibration fluid (C),
- the measured value ($U_{pH}(C')$) corresponding to the pH value (pH(C')) of the calibration fluid (C') produced from the predetermined calibration fluid (C), as well as
- the measured value ($U_{pH}(BF)$) corresponding to the pH value (pH(BF)) of the baseline fluid (BF) are determined and
- with these determined measured values ($U_{pCO2}(C)$, $U_{pCO2}(C')$, $U_{pH}(C)$, $U_{pH}(C')$ and $U_{pH}(BF)$) and with the defined pH values (pH(C) and pH (BF)) of the predetermined calibration fluid (C) and baseline fluid (BF) itself, the variable

$$S_{pCO2} = K_{pCO2} \cdot S_{pH}$$

is formed with

$$K_{pCO2} = -[(U_{pCO2}(C) - U_{pCO2}(C'))/(U_{pH}(C) - U_{pH}(C'))]$$

and

$$S_{pH} = (\Delta U_{pH}(C))/(pH(C) - pH(BF))$$

with

$$\Delta U_{pH}(C) = U_{pH}(C) - U_{pH}(BF),$$

by means of which variable $S_{pCO2}$
- the $CO_2$ partial pressure ($pCO_2$(B)) of $CO_2$ dissolved in the blood (B) can be determined using
- a measured value ($U_{PCO2}$(B)) that is determined by supplying blood (B) to the $CO_2$ sensor (1) and that corresponds to the $CO_2$ partial pressure ($pCO_2$(B)) of the blood (B) and
- a measured value ($U_{pCO2}$ (BF)) that is determined by supplying baseline fluid (BF) to the $CO_2$ sensor (1) and that corresponds to the $CO_2$ partial pressure ($pCO_2$(BF)) of the baseline fluid (BF) on the basis of

$$\log(pCO2(B)) = (\Delta U_{pCO2}(B) - \Delta U_{pCO2}(C))/S_{pCO2} + \log(pCO_2(C))$$

wherein

$$\Delta U_{pCO2}(B) = U_{pCO2}(B) - U_{pCO2}(BF)$$

and

$$\Delta U_{pCO2}(C) = U_{pCO2}(C) - U_{pCO2}(BF).$$

16. A method according to claim 15,
**characterised**
**in that** the $CO_2$ partial pressure ($pCO2$(BF)) of the baseline fluid (BF) changes over the course of time (t) and in that

- the variable $\Delta U_{pCO2}$(C) is formed by

$$\Delta U_{pCO2}(C) = U_{pCO2}(C) - U_{pCO2}(BF(t_c))$$

with a measured value ($U_{pCO2}$(BF($t_c$)) of the $CO_2$ partial pressure ($pCO_2$(BF)) of the baseline fluid (BF) measured close in time to the $CO_2$ measurement undertaken on the predetermined calibration fluid (C) and
- the variable $\Delta U_{pCO2}$(B) is formed by

$$\Delta U_{pCO2}(B) = U_{pCO2}(B) - U_{pCO2}(BF(t_b))$$

with a measured value ($Up_{CO2}$(BF($t_b$)) of the $CO_2$ partial pressure ($pCO_2$ (BF)) of the baseline fluid (BF) measured close in time to the $CO_2$ measurement undertaken on the blood (B).

17. A process according to claim 15 or claim 16,
**characterised in that**
a predetermined calibration fluid (C) is used in which $NaHCO_3$ is dissolved in a physiological concentration.

18. A process according to any one of claims 15 to 17,
**characterised in that**
the calibration fluid (C') produced from the predetermined calibration fluid (C) is produced by allowing $CO_2$ temporarily to outgas from the predetermined calibration fluid (C).

19. A method according to claim 18,
**characterised**
**in that** the predetermined calibration fluid (C) is supplied to the sensors (1, 2) through a tube (16) having a gas-permeable tube wall (160) surrounded by the gas phase (Gph) and an internal cross-section (161) which, relative to a quantity of predetermined calibration fluid (C) to be provided in a determined unit of time to the sensors (1, 2), is of a sufficiently small size that, while being supplied to the sensors (1, 2), a calibration fluid (C, C') does not undergo a change going beyond a specifiable, allowed extent in the concentration (pCO2(C)) of the $CO_2$ dissolved in this fluid (C), and

**in that** the calibration fluid (C') of the predetermined other concentration (pCO$_2$(C')) of $CO_2$ which is to be produced from the predetermined calibration fluid (C) is produced by allowing the predetermined calibration fluid (C) to dwell temporarily in the tube (16) and material transport between this fluid (C) and the gas phase (Gph) through the tube wall (160).

20. A method according to any one of claims 15 to 19,
   **characterised in that**
   a baseline fluid (BF) which is air-saturated under normal conditions is used.

21. A method according to any one of claims 16 to 20,
   **characterised in that**
   the baseline fluid (BF) is supplied to the sensors (1, 2) through a gas-permeable tube (12) having an internal cross-section (121) which, relative to a quantity of baseline fluid (BF) to be provided in a determined unit of time to the sensors (1, 2), is of a sufficiently large size that, while being supplied to the sensors (1, 2), the baseline fluid (BF) remains in the tube (12) for a dwell time which is sufficient for the baseline fluid (BF) to adapt to an ambient pressure.

22. A method according to any one of claims 15 to 21,
   **characterised in that**
   the baseline fluid (BF) is taken from a gas-impermeable vessel (15).

23. A method according to any one of claims 15 to 22,
   **characterised in that**
   a variable $S_{pCO2}$ which is determined once is used for the repeated determination of the $CO_2$ partial pressure (pCO$_2$(B)) of blood (B) by in each case determining the measured value ($U_{pCO2}$(B)) corresponding to the $CO_2$ partial pressure (pCO$_2$(B)) of the blood (B) and the measured value ($U_{pCO2}$(BF)) corresponding to the $CO_2$ partial pressure (pCO$_2$(BF)) of the baseline fluid (BF).

**Revendications**

1. Procédé pour étalonner un capteur (1) servant à mesurer la concentration (pG) d'un gaz (G) dissous dans un fluide (B, C, C') et à générer une valeur mesurée ($U_{pG}$ ; $I_{pG}$) correspondant à la concentration mesurée (pG), le capteur (1) étant approvisionné pour étalonnage avec :

   - au moins deux fluides d'étalonnage (C, C') par un tube (16), le tube (16) ayant une paroi de tube perméable aux gaz (160), chacun des fluides d'étalonnage contenant le gaz (G) dans une concentration de dissolution définie avec précision mais différente (pG(C), pG(C')) et
   - les valeurs mesurées ($U_{pG}$(C), $U_{pG}$(C') ; $I_{pG}$(C), $I_{pG}$(C')) correspondant à ces différentes concentrations (pG(C), pG(C')) étant déterminées,

   **caractérisé**
   **en ce qu'**au moins un des deux fluides d'étalonnage est produit à partir d'un fluide d'étalonnage prédéterminé en faisant passer la concentration (pG(C)) du gaz (G) dissous dans ce fluide d'étalonnage prédéterminé (C) à une autre concentration prédéterminée (pG(C')) de ce gaz (G) et
   **en ce que** le passage de la concentration (pG(C)) du gaz (G) dissous dans ce fluide d'étalonnage prédéterminé (C) à l'autre concentration prédéterminée (pG(C')) est produit par transport matériel à travers la paroi de tube perméable aux gaz (160) entre le fluide d'étalonnage prédéterminé (C) et une phase gazeuse prédéterminée (Gph) .

2. Procédé selon la revendication 1, **caractérisé en ce qu'**une phase gazeuse (Gph) est utilisée, qui contient le gaz (G) avec une pression partielle (pG(Gph)) qui diffère de la pression partielle (pG(C) du gaz (G) dissous dans le fluide d'étalonnage prédéterminé (C), de telle sorte qu'il existe une différence de pression partielle (ΔpG(Cph, G)) en ce qui concerne le gaz (G) entre la phase gazeuse (Gph) et le fluide d'étalonnage prédéterminé (C), et **en ce qu'**un procédé d'égalisation de cette différence de pression (ΔpG (A, G)), piloté par cette différence de pression, partielle (ΔpG (A, G)), est maintenu jusqu'à ce qu'un fluide d'étalonnage (C') ayant une pression partielle (pG(C')) de ce gaz (G) qui corresponde à l'autre concentration prédéterminée soit obtenu.

3. Procédé selon la revendication 1 ou la revendication 2, **caractérisé en ce qu'**une phase gazeuse (Gph) constituée d'air est utilisée.

4. Procédé selon l'une quelconque des revendications 1 à 3, **caractérisé en ce que** le fluide d'étalonnage prédéterminé (C) est fourni au capteur (1) par un tube (16) ayant une paroi de tube perméable aux gaz (160) entourée par la phase gazeuse (Gph) et une section transversale interne (161) qui, en ce qui concerne une quantité de fluide d'étalonnage prédéterminé (C) à fournir dans une unité de temps déterminée au capteur (1), est de taille suffisamment petite pour que, lorsqu'il est fourni au capteur (1), un fluide d'étalonnage (C, C') ne subit pas de changement au-delà d'une limite autorisée spécifiable de la concentration (pG(C)) du gaz (C) dissous dans ce fluide (C) à cause du transport matériel entre ce fluide (C) et la phase gazeuse (Gph) à travers la paroi de tube (160) et, **en ce que** le fluide d'étalonnage (C') de l'autre concentration prédéterminée (pG(C')) qui doit être produit à partir du fluide d'étalonnage prédéterminé (C) est produit en autorisant le fluide d'étalonnage prédéterminé (C) à séjourner temporairement dans le tube (3) et le transport matériel entre ce fluide (C) et la phase gazeuse (Gph) à travers la paroi de tube (3).

5. Procédé selon la revendication 4, **caractérisé en ce que** le fluide d'étalonnage prédéterminé (C) est prélevé dans un récipient imperméable aux gaz (18).

6. Procédé selon l'une quelconque des revendications précédentes, **caractérisé en ce que** le capteur (1) génère une valeur mesurée ($I_{kG}(B)$, $I_{kG}(C)$, $I_{kG}(C')$) qui est directement proportionnelle à la concentration (pG) du gaz (C) dissous dans le fluide (B, C, C') fourni au capteur (1).

7. Procédé selon l'une quelconque des revendications 1 à 5, **caractérisé en ce que** le capteur (1) génère une valeur mesurée ($U_{kG}(B)$, $U_{kG}(C)$, $U_{kG}(C')$) qui est proportionnelle au logarithme de la concentration (pG) du gaz (C) dissous dans le fluide (B, C, C') fourni au capteur (1).

8. Procédé selon l'une quelconque des revendications précédentes, en particulier selon la revendication 7, **caractérisé en ce qu'**un fluide d'étalonnage prédéterminé (C) est utilisé, dans lequel la concentration (pG(C)) du gaz (G) dissous dans ce fluide d'étalonnage (C) est une fonction définie non constante (f) de la valeur de pH variable (pH(C)) de ce fluide d'étalonnage, et
**en ce que** l'autre concentration prédéterminée (pG(C')) du fluide d'étalonnage (C') produit par le fluide d'étalonnage prédéterminé (C) est déterminée avec précision par l'intermédiaire de la valeur de pH (pH(C')) du fluide d'étalonnage (C') produit de cette manière.

9. Procédé selon la revendication 8, **caractérisé en ce qu'**un fluide d'étalonnage prédéterminé (C) est utilisé, dans lequel le logarithme de la concentration (pG(C)) du gaz (G) dissous dans ce fluide d'étalonnage (C) est une fonction linéaire non constante de la valeur de pH variable (pH) de ce fluide d'étalonnage (C).

10. Procédé selon la revendication 7 et la revendication 9, **caractérisé en ce que** le fluide d'étalonnage prédéterminé (C) et le fluide d'étalonnage (C') de l'autre concentration prédéterminée (pG(C')) produit à partir de celui-ci est fourni à un capteur de pH (2) servant à mesurer la valeur de pH et à générer une valeur mesurée ($U_{pH}$) proportionnelle à la valeur de pH (pH) et les valeurs mesurées ($U_{pH}(C)$, $U_{pH}(C')$) correspondant aux valeurs de pH différentes de ces deux fluides d'étalonnage (C, C') sont déterminées, et
**en ce que** la valeur

$$-K_{pG} = (U_{pG}(C) - (U_{pG}(C'))) / (U_{pH}(C) - (U_{pH}(C')))$$

est déterminée à partir des valeurs mesurées ($U_{pG}(C)$, $U_{pG}(C')$) déterminées par un capteur (1) et les valeurs mesurées ($U_{pH}(C)$, $U_{pH}(C')$) déterminées par le capteur de pH (2).

11. Procédé selon l'une quelconque des revendications précédentes, **caractérisé en ce qu'**un capteur de $CO_2$ (1) servant à mesurer la pression partielle ($pCO_2$) du $CO_2$ dissous dans un fluide (B) est utilisé comme capteur (1) pour mesurer la concentration d'un gaz (G) dissous dans un fluide (B, C, C').

**12.** Procédé selon l'une quelconque des revendications précédentes, **caractérisé en ce qu'**un capteur pour mesurer la pression partielle (pG) d'un gaz (G) dissous dans un fluide corporel physiologique (B) et/ou dans un tissu physiologique est utilisé comme capteur (1) pour mesurer la concentration d'un gaz (G) dissous dans un fluide (B, C, C').

**13.** Procédé selon la revendication 11 et la revendication 12, **caractérisé en ce qu'**un capteur de $CO_2$ (1) est utilisé pour mesurer la pression partielle ($pCO_2$) de $CO_2$ dissous dans le sang (B).

**14.** Procédé selon les revendications 9 et 11 ou 13, **caractérisé en ce qu'**un fluide d'étalonnage prédéterminé (C) est utilisé, dans lequel du $HCO_3^-$ est dissous.

**15.** Procédé pour mesurer la pression partielle ($pCO_2(B)$) de $CO_2$ dissous dans le sang (B) avec un capteur de $CO_2$ (1) servant à mesurer la pression partielle de $CO_2$ ($pCO_2$) et générer une valeur mesurée ($U_{pCO_2}$) proportionnelle au logarithme de la pression partielle de $CO_2$ ($pCO_2$) au moyen d'un procédé selon les revendications 10 et 13 et 14, **caractérisé en ce que** successivement, dans une séquence arbitraire,

- le capteur de $CO_2$ (1) et le capteur de pH (2) sont approvisionnés avec
- le fluide d'étalonnage prédéterminé (C) dans lequel le $CO_2$ est dissous avec une pression partielle physiologique $pCO_2(C)$ définie avec précision et dans lequel du $HCO_3^-$ est dissous avec une concentration physiologique ($cHCO_3^-$) définie avec précision et qui a une valeur de pH physiologique (pH(C)) définie avec précision, et
- le fluide d'étalonnage (C') produit à partir du fluide d'étalonnage prédéterminé (C) par transport matériel entre ce fluide, (C) et la phase gazeuse prédéterminée (Gph), ce fluide d'étalonnage (C') ne différant du fluide d'étalonnage prédéterminé (C) que sur la base de la pression partielle de $CO_2$ physiologique $pCO_2(C')$), qui diffère de la pression partielle de $CO_2$ ($pCO_2(C)$) du fluide d'étalonnage prédéterminé (C), et que sur la base de la valeur de pH physiologique (pH(C')) déterminée uniquement ainsi qui diffère de la valeur de pH (pH(C)) du fluide d'étalonnage prédéterminé (C), et
- le capteur de pH (2) est également approvisionné avec
- un fluide de base (BF) dans lequel du $CO_2$ est dissous avec une pression partielle $pCO_2(BF)$) de moins de 10 Torr et qui a une valeur de pH physiologique (pH(BF)) définie différente de la valeur de pH (pH(C)) du fluide d'étalonnage prédéterminé (C), et
- la valeur mesurée ($U_{pCO_2}(C)$) correspondant à la pression partielle de $CO_2$ ($pCO_2(C)$) du fluide d'étalonnage prédéterminé (C),
- la valeur mesurée ($U_{pCO_2}(C')$) correspondant à la pression partielle de $CO_2$ ($pCO_2(C')$) du fluide d'étalonnage (C') produit par le fluide d'étalonnage prédéterminé (C),
- la valeur mesurée ($U_{pH}(C)$) correspondant à la valeur de pH (pH(C)) du fluide d'étalonnage prédéterminé (C),
- la valeur mesurée ($U_{pH}(C')$) correspondant à la valeur de pH (pH(C')) du fluide d'étalonnage (C') produit à partir du fluide d'étalonnage prédéterminé (C), ainsi que
- la valeur mesurée ($U_{pH}(BF)$) correspondant à la valeur de pH (pH(BF).) du fluide de base (BF) sont déterminées et ,
- avec ces valeurs mesurées déterminées ($U_{pCO_2}(C)$, $U_{pCO_2}(C')$, $U_{pH}(C)$, $U_{pH}(C')$ et $U_{pH}(BF)$) et avec les valeurs de pH définies (pH (C) et pH(BF)) du fluide d'étalonnage prédéterminé (C) et du fluide de base (BF) lui-même, la grandeur

$$S_{pCO2} = K_{pCO2} \cdot S_{pH}$$

est déterminée par

$$K_{pCO2} = - [(U_{pCO2}(C) - U_{pCO2}(C')) / (U_{pH}(C) - (U_{pH}(C')))]$$

et

$$S_{pH} = (\Delta U_{pH}(C)) / (pH(C) - pH(BF))$$

avec

$$\Delta U_{pH}(C) = U_{pH}(C) - U_{pH}(BF),$$

au moyen de laquelle grandeur $S_{pCO2}$
- la pression partielle de $CO_2$ ($pCO_2(B)$) du $CO_2$ dissous dans le sang (B) peut être déterminée avec
- une valeur mesurée ($U_{pCO2}(B)$) qui est déterminée en fournissant du sang (B) au capteur de $CO_2$ (1) et qui correspond à la pression partielle de $CO_2$ ($pCO_2(B)$) du sang (B) et
- une valeur mesurée ($U_{pCO2}(BF)$) qui est déterminée en fournissant du fluide de base (BF) au capteur de $CO_2$ (1) et qui correspond à la pression partielle de $CO_2$ ($pCO_2(BE)$) du fluide de base (BF) d'après la relation

$$\log(pCO_2(B)) = (\Delta U_{pCO2}(B) - \Delta U_{pCO2}(C))/S_{pCO2} + \log(pCO_2(C))$$

dans laquelle

$$\Delta U_{pCO2}(B) = U_{pCO2}(B) - U_{pCO2}(BF)$$

et

$$\Delta U_{pCO2}(C) = U_{pCO2}(C) - U_{pCO2}(BF).$$

**16.** Procédé selon la revendication 15, **caractérisé en ce que** la pression partielle de $CO_2$ ($pCO_2(BF)$) du fluide de base (BF) change au cours du temps (t) et **en ce que**

- la variable $\Delta U_{pCO2}(C)$) est déterminée par

$$\Delta U_{pCO2}(C) = U_{pCO2}(C) - U_{pCO2}(BF(t_c))$$

avec une valeur mesurée ($U_{pCO2}(BF(t_c))$ de la pression partielle de $CO_2$ ($pCO_2(BF)$) du fluide de base (BF) mesurée à un instant proche de celui de la mesure de $CO_2$ effectuée sur le fluide d'étalonnage prédéterminé (C) et
- la variable $\Delta U_{pCO2}(B)$ est déterminée par

$$\Delta U_{pCO2}(B) = U_{pCO2}(B) - U_{pCO2}(BF(t_b))$$

avec une valeur mesurée ($U_{pCO2}(BF(t_b))$) de la pression partielle de $CO_2$ ($pCO_2(BF)$) du fluide de base (BF) mesurée à un instant proche de celui de la mesure de $CO_2$ effectuée sur le sang (B).

**17.** Procédé selon la revendication 15 ou la revendication 16, **caractérisé en ce qu'**un fluide d'étalonnage prédéterminé (C) est utilisé dans lequel du $NaHCO_3$ est dissous à une concentration physiologique.

**18.** Procédé selon l'une quelconque des revendications 15 à 17, **caractérisé en ce que** le fluide d'étalonnage (C') produit à partir du fluide d'étalonnage prédéterminé (C) est produit en permettant temporairement au $CO_2$ de s'échapper du fluide d'étalonnage prédéterminé (C).

**19.** Procédé selon la revendication 18, **caractérisé en ce que** le fluide d'étalonnage prédéterminé (C) est fourni aux capteurs (1, 2) par un tube (16) ayant une paroi de tube perméable aux gaz (160) entourée par la phase gazeuse (Gph) et une section transversale interne (161) qui, en ce qui concerne une quantité de fluide d'étalonnage prédéterminé (C) à fournir dans une unité de temps déterminée aux capteurs (1, 2), est de taille suffisamment petite pour que, lorsqu'il est fourni aux capteurs (1, 2), un fluide d'étalonnage (C, C') ne subisse pas de changement au-delà d'une limite autorisée spécifiable de la concentration (pCO$_2$(C)) du CO$_2$ dissous dans ce fluide (C), et **en ce que** le fluide d'étalonnage (C') de l'autre concentration prédéterminée (pCO$_2$(C')) de CO$_2$, qui doit être produit à partir du fluide d'étalonnage prédéterminé (C), est produit en permettant au fluide d'étalonnage prédéterminé (C) de séjourner temporairement dans le tube (16) et le transport matériel entre ce fluide (C) et la phase gazeuse (Gph) à travers la paroi de tube (160).

20. Procédé selon l'une quelconque des revendications 15 à 19, **caractérisé en ce qu'**un fluide de base (BF) qui est saturé d'air dans les conditions normales est utilisé.

21. Procédé selon l'une quelconque des revendications 16 à 20, **caractérisé en ce que** le fluide de base (BF) est fourni aux capteurs (1, 2) par un tube perméable aux gaz (12) ayant une section transversale interne (121) qui, en ce qui concerne une quantité de fluide de base (BF) à fournir dans une unité de temps déterminée aux capteurs (1, 2), est d'une taille suffisamment importante pour que, lorsqu'il est fourni aux capteurs (1, 2), le fluide de base (BF) reste dans le tube (12) pendant une durée de séjour qui est suffisante pour permettre au fluide de base (BF) de s'adapter à une pression ambiante.

22. Procédé selon l'une quelconque des revendications 15 à 21, **caractérisé en ce que** le fluide de base (BF) est prélevé dans un récipient imperméable aux gaz.

23. Procédé selon l'une quelconque des revendications 15 à 22, **caractérisé en ce qu'**une variable S$_{pCO2}$ qui est déterminée une fois est utilisée pour la détermination répétée de la pression partielle de CO$_2$ (pCO$_2$(B)) du sang (B) par la détermination, dans chaque cas, de la valeur mesurée (U$_{pCO2}$(B)) correspondant à la pression partielle de CO$_2$ (pCO$_2$(B)) du sang (B) et de la valeur mesurée (U$_{pCO2}$(BF)) correspondant à la pression partielle de CO$_2$ (pCO$_2$(BF)) du fluide de base (BF).

FIG 1

FIG 2

FIG 3

FIG 4

FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4221567 A